# EUROPEAN PATENT SPECIFICATION

(11) **EP 2 476 422 B1**
(45) Date of publication and mention of the grant of the patent: **16.03.2016**
(21) Application number: 09848864.6
(22) Date of filing: 07.09.2009
(51) Int. Cl.: A61K 31/704, A61K 31/58, A61P 7/02, A61P 3/10, A61P 9/10, A61K 31/7048, A61K 36/8964

(54) **PHARMACEUTICAL COMPOSITON COMPRISING TIMOSAPONIN A-III AND TIMOSAPONIN B-II**
PHARMAZEUTISCHE ZUSAMMENSETZUNG ENTHALTEND TIMOSAPONIN A-III AND TIMOSAPONIN B-II ZUR BEHANDLUNG VON THROMBOTISCHEN ERKRANKUNGEN
COMPOSITIONS PHARMACEUTIQUES CONTENANT DU TIMOSAPONIN A-III ET DU TIMOSAPONIN B-II DESTINEES AU TRAITEMENT DES MALADIES THROMBOTIQUES

(43) Date of publication of application: 18.07.2012
(73) Proprietor: Institute of Radiation Medicine, Academy of Military Medical Sciences, PLA, Beijing 100850 (CN)
(72) Inventor: MA, Baiping, Beijing 100850 (CN); CONG, Yuwen, Beijing 100850 (CN); KANG, Liping, Beijing 100850 (CN); GAO, Yue, Beijing 100850 (CN); TAN, Dawei, Beijing 100850 (CN); XIONG, Chengqi, Beijing 100850 (CN); ZHAO, Yang, Beijing 100850 (CN)
(74) Representative: Kling, Simone
(86) International application number: PCT/CN2009/001009
(87) International publication number: WO 2011/026259

(56) References cited:
- EP-A1- 1 741 434
- WO-A2-2009/151762
- CN-A- 1 451 384
- CN-A- 1 692 908
- CN-A- 101 658 525
- CN-C- 1 199 643

## Description

The present invention relates to a pharmaceutical composition for combating thrombotic a disease, a method for preparing the same, and uses thereof, particularly to a pharmaceutical composition comprising timosaponin AIII and timosaponin BII, a method for preparing the same, and uses thereof for manufacturing a medicament for the prophylaxis or treatment of a thrombotic disease.

### BACKGROUND ART

The adhesion, aggregation and release reaction of platelets lead to thrombosis. Thrombosis is a main pathogen for many human cerebrovascular diseases such as myocardial infarction, cerebral apoplexy, etc., and a aggravation factor for some important diseases such as diabetes and vasculitis. Antithrombotic therapy is one of the main therapies for these diseases. Inhibition of platelet aggregation, anticoagulation, and thrombolysis are three main topics for treatment of a thrombotic disease, in which anti-platelet aggregation is the most popular therapy with prominent therapeutical effect, and the research and development of relevant drugs are the most active. Many drugs, from aspirin as the earliest cyclooxygenase inhibitor, to ticlopidine as ADP receptor antagonist, to tirofiban as platelet GPIIb/IIIa receptor antagonist, as well as serial prostaglandins such as PGE1, PGI2 and so on are wildly used for clinical treatment of cardiac and cerebral vascular diseases.

Chinese herb *Rhizome anemarrhenae* (Zhi Mu) is the rootstock of herbaceous perennial *Anemarrhena asphodeloides Bge.* of Anemarrhena Bunge of Liliaceae, which main active components are steroidal saponins. So far, several dozens of steroidal saponins and sapogenins separated and identified from Rhizome anemarrhenae, as well as flavones, oligosaccharides, polysaccharides, fatty acids and so on have been reported in documents.

Jianying ZHANG et al reported that single compounds such as timosaponins la, BI, BII, BIII and AIII had significant activity against human platelet aggregation and extending blood coagulation time (Jianying ZHANG, et al, Clinica Chimica Acta, 1999; 289: 79-88).

Timosaponins AIII, B and BII have the following structures:

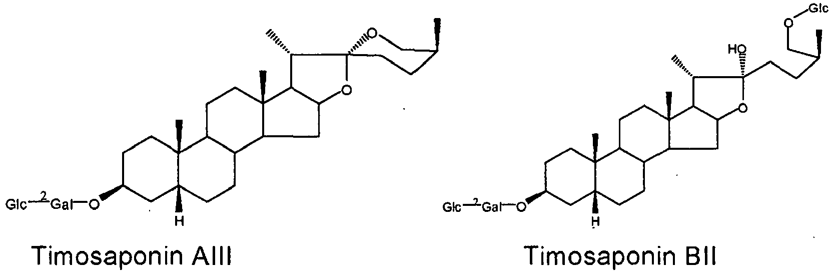

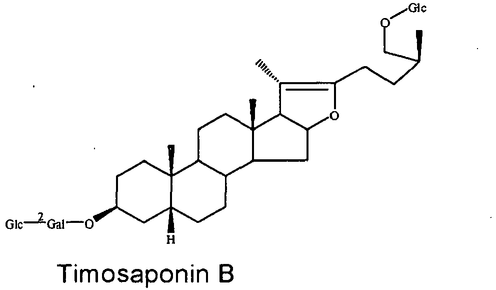

Baiping MA, et al reported single compound timosaponin BII could significantly improve nvervous symptoms of rats with cerebral ischemia, reduce cerebral infarction area, and alleviate cerebral edema; could significantly improve blood rheology, alleviate inflammatory injury caused by cerebral ischemia; and could be used for prevention of cerebral apoplexy (stroke) (Chinese patent application No.: 200410037347.X).

Wansheng CHEN, et al reported the use of total timosaponins for preparing medicaments for prophylaxis and treatment of cerebral apoplexy (Chinese patent application publication No. CN1451384A with the application No. 03116824.8). In this application, the disclosed total timosaponins are featured that the sum of contents of timosaponins BII, E, B, AIII is ≥50%.

Wansheng CHEN, et al further reported that the injection of extract of Rhizome anemarrhenae (containing timosaponins BII, E1 and B, the ratio thereof is 78-92: 7-12: 0-6) can significantly improve behavior symptoms caused by ischemia reperfusion injury in rats, reduce cerebral infarction area, alleviate cerebral edema in cerebral ischemia rats, and can be used for treatment of ischemic cerebrovascular diseases (Chinese patent application publication No. CN1628790A with the application No. 200410054146.0).

So far, an antithrombotic drug comprising timosaponin AIII as the main active component in combination with timosaponin BII has not been found. Hence, it will meet the clinical need to provide an antithrombotic drug comprising timosaponin AIII as the main active component in combination with timosaponin BII.

### DESCRIPTION OF THE INVENTION

One object of the present invention is to provide a pharmaceutical composition mainly comprising timosaponin AIII and timosaponin BII for the prophylaxis or treatment of a thrombotic disease.

Another object of the present invention is to provide a method for preparing the pharmaceutical composition of the present invention.

A further another object of the present invention is to provide a use of the pharmaceutical composition of the present invention in manufacturing a medicament for the prophylaxis or treatment of a thrombotic disease.

After intensively investigating for several years, the inventors of the present invention have first found and confirmed that a combination of timosaponin AIII and timosaponin BII is used for combating a thrombotic disease, so long as the amount of timosaponin AIII is greater than or equal to the amount of timosaponin BII, such combination can not only produce a satisfactory antithrombotic effect, but also alleviate the bleeding or bleeding tendency that usually occurs in the use of antithrombotic agents. Based upon this finding, the present inventors have completed the present invention.

In one aspect, the present invention provides a pharmaceutical composition for the prophylaxis or treatment of a thrombotic disease, comprising an effective amount of timosaponin AIII and timosaponin BII, and optionally one or more pharmaceutically acceptable excipients, characterized in that the amount of timosaponin AIII is greater than or equal to the amount of timosaponin BII.

In one embodiment, both timosaponin AIII and timosaponin BII are used in the pharmaceutical composition of the present invention in the form of timosaponin extracts. In this embodiment, the pharmaceutical composition may only comprise an effective amount of an extract comprising timosaponin AIII and an extract comprising timosaponin BII, and comprise no pharmaceutically acceptable excipients.

In another embodiment, the weight ratio of timosaponin AIII to timosaponin BII is 1:1 to 10:1 in the pharmaceutical composition of the present invention.

In another embodiment, the weight ratio of timosaponin AIII to timosaponin BII is 2:1 to 5:1 in the pharmaceutical composition of the present invention.

In another embodiment, the weight ratio of timosaponin AIII to timosaponin BII is 3:1 in the pharmaceutical composition of the present invention.

In another aspect, the present invention provides a method for preparing the pharmaceutical composition of the present invention, comprising mixing an extract containing the desired amount of timosaponin AIII and an extract containing the desired amount of timosaponin BII, and optionally adding one or more pharmaceutically acceptable excipients, depending on the needs, and then formulating the mixture into a desired preparation by a suitable method.

In one embodiment, the method for preparing the pharmaceutical composition of the present invention comprises mixing the desired amount of timosaponin AIII compound and timosaponin BII compound, together with one or more pharmaceutically acceptable excipients, and then formulating the mixture into a desired preparation by a suitable method.

In another embodiment, the method for preparing the pharmaceutical composition of the present invention comprises the following steps:
extracting decoction pieces, fresh rootstock, fibrous root and so on, of *Rhizome anemarrhenae* by using a suitable extraction method, filtering the resultant extracting solution, collecting the filtrate, then loading the filtrate into a macroporous adsorbent resin column, eluting with a suitable solvent, collecting a corresponding component, and separating it to obtain primary total saponins of *Rhizome anemarrhenae* mainly comprising timosaponin BII;
transforming the components by using natural fermentation, enzymatic conversion, buffer salt conversion, acid hydrolysis or a combination thereof for a sufficient period to obtain secondary total saponins of *Rhizome anemarrhenae;* and
mixing the primary total saponins and the secondary total saponins of *Rhizome anemarrhenae* in a certain ratio to obtain the pharmaceutical composition of the present invention.

In one specific embodiment, the method for preparing the pharmaceutical composition of the present invention comprises the following steps:
extracting decoction pieces, fresh rootstock or fibrous root of *Rhizome anemarrhenae* with 40-70% a C₁-C₄ alcohol or 40-70% acetone, filtering the resultant extracting solution, collecting and centrifuging the filtrate, then loading the supernatant into a macroporous adsorbent resin column, eluting with a solvent selected from the group consisting of water, 20-90% a C₁-C₄ alcohol and 10-80% acetone in a gradient, and collecting a 50-90% a C₁-C₄ alcohol component or 35-80% acetone component to obtain primary total saponins of *Rhizome anemarrhenae;*
transforming the components with an enzyme selected from the group consisting of β-glucanase, β-glucosidase, pectinase, cellulase, emulsin and *Aspergillus niger* or a microorganism for a sufficient period, and centrifuging conversion liquid to obtain secondary total saponins of *Rhizome anemarrhenae;* and
mixing the primary total saponins and the secondary total saponins of *Rhizome anemarrhenae* in a certain ratio to obtain the pharmaceutical composition of the present invention.

In one specific embodiment, the method for preparing the pharmaceutical composition of the present invention comprises the following steps:
extracting decoction pieces, fresh rootstock or fibrous root of *Rhizome anemarrhenae* with 40-70% ethanol, filtering the resultant extracting solution and collecting the filtrate, concentrating under a reduced pressure and then adding with 90-100% ethanol, centrifuging , then loading the supernatant into a macroporous adsorbent resin column, eluting with 20-95% ethanol in a gradient, and collecting a 50-90% ethanol component to obtain primary total saponins of *Rhizome anemarrhenae;*
transforming the components with one or more enzymes selected from the group consisting of β-glucanase, β-glucosidase, pectinase, cellulase, emulsin and *Aspergillus niger* or a microorganism for at least one hour, and centrifuging the conversion liquid to obtain secondary total saponins of *Rhizome anemarrhenae;* and
mixing the primary total saponins and the secondary total saponins of *Rhizome anemarrhenae* in a certain ratio to obtain the pharmaceutical composition of the present invention.

In another aspect, the present invention provides a use of timosaponin AIII and timosaponin BII in manufacturing a medicament for the prophylaxis or treatment of a thrombotic disease, in which the amount of timosaponin AIII is greater than or equal to the amount of timosaponin BII in the manufactured medicament.

In one embodiment, the weight ratio of timosaponin AIII to timosaponin BII is 1:1 to 10:1 in the manufactured medicament.

In another embodiment, the weight ratio of timosaponin AIII to timosaponin BII is 2:1 to 5:1 in the manufactured medicament.

In another embodiment, the weight ratio of timosaponin AIII to timosaponin BII is 3:1 in the manufactured medicament.

In another embodiment, the a thrombotic disease or thrombus associated diseases comprise, for example, coronary heart disease, angina, myocardial infarction, cerebral apoplexy, cerebral thromobosis, pulmonary embolism, diabetes and vasculitis.

The term "an effective amount" used herein refers to the amount of timosaponin AIII and timosaponin BII in combination that can achieve the clinically prophylaxis or treatment of a thrombotic disease.

As for those skilled in the art, the pharmaceutical composition of the present invention can be readily formulated by various conventional methods in the art into various kinds of dosage forms, such as oral preparations, such as tablets, capsules, solutions, suspensions and granules; dosage forms for parenteral administration, such as injections, ointments, patches. The pharmaceutical composition of the present invention can be used via various administration routes in the art.

The pharmaceutical composition or preparations thereof can be provided to a patient in need thereof by oral administration or parenteral administration. For an adult, its daily oral dose for one person is 150mg to 450mg, for example, about 300mg.

It had been shown by investigating that timosaponin AIII could significantly inhibit platelet aggregation in vitro, improve the effect of PGE1 for combating platelet aggregation, and have significant antithrombotic acitivity; while timosaponin BII could nto inhibit platelet aggregation in vitro, but could expand blood vessels, improve blood rheology in vivo, and reduce the adhesion of leukocytes on vascular endothelial cells. Hence, in the present invention, timosaponin AIII and timosaponin BII which have different mechanisms of action and targets are combined in a certain ratio, and thus can obtain a potent antithrombotic effect with less bleeding tendency in vivo.

Hence, the pharmaceutical composition of the present invention can not only achieve the prophylaxis or treatment of a thrombotic disease, but also alleviate the bleeding or bleeding tendency in a patient.

### BRIEF DESCRIPTION OF THE DRAWINGS

Fig.1 is a scatter diagram of the lethal time by caudal vein injection of collagen in various groups of mice.
Fig.2 is a scatter diagram of bleeding time in caudal vein in various groups of mice.

### EXAMPLES FOR CARRYING OUT THE INVENTION

The following examples are used to further illustrating the present invention.

Unless specifically indicated otherwise, the percentages or parts in the text refer to weight percentages or parts, and the sum of contents of components in composition is equal to 100%.

### Example 1

### Effects of the present pharmaceutical composition on bleeding time in caudal vein and lethal time by caudal vein injection of collagen in mice

### I. Experimental materials and methods

### 1. Experimental materials

Timosaponins AIII and BII single compounds were prepared according to known methods (Acta Pharmaceutica Sinica, 1996; 31(4): 271-277; Chem Pharm Bull, 1963, 11: 1221), and both of these two compounds had purity of greater than 98.5%; Wistar rats, male, body weight 280-320g, Kunming mice, male, body weight 22-24g, were provided by the Animal Breeding Center of the Academy of Military Medical Sciences. Adrenaline was purchased from Beijing Yongkang Pharmaceutical Co., Ltd. Collagen was self-made rat tail collagen. PBS was purchased from Tianweishidai Company. Heparin was purchased from Sigma Company. Physiological saline solution was purchased from Shandong Zibo Pharmaceutical Factory. Aspirin was from Ouyi Pharmaceutical Company of Shijiazhuang Pharmaceutical Group Ltd.

### 2. Animal grouping

Wistar rats were randomly divided into 7 groups: control group, aspirin group (ASP,40mg/Kg), AIII group (40 mg/Kg), BII group (40 mg/Kg), 1:1 group (weight ratio of AIII to BII, 40 mg/Kg), 1:3 group (weight ratio of AIII to BII, 40 mg/Kg), 3:1 group (weight ratio of AIII to BII, 40 mg/Kg). Continuous gastric administration was performed for 7 days, once per day, and the experiments were conducted after 1 h of the administration on the 7^{th} day. The control group was administered with physiological saline of the same volume.

### 3. Experimental methods

### 3.1 Detection of maximum aggregation rate of platelet

Wistar rats, after 1 h of administration on the 7^{th} day, were anesthetized by intraperitoneal injection of pentobarbital sodium (40-60 mg/kg), blood samples were collected from hearts, anti-coagulated with 3.8% sodium citrate (volume ratio: 1:9). Centrifugation was performed under 800rpm and room temperature for 10min to separate platelet-rich plasmas (PRP), then centrifugation was performed again under 3000rpm and room temperature for 20min to separate platelet-poor plasmas (PPP). Counting was performed by using F-820 blood cell counter, and PRP was adjusted with PPP to have a concentration of 3.0×10¹¹/L.

Chronolog platelet aggregation analyzer test: Platelet suspension was adjusted with plasma to have a concentration of 3.0×10¹¹/L, the platelet aggregation analyzer was turned on and pre-warmed for 30min, and PPP was used as a blank control to adjust transmittance to be 100%. 450µL of platelet suspension were taken, stirred with a stir bar and pre-warmed at 37°C for 3min, and separately added with inducing agent (50µL) ADP agent (final concentration: 20 µM), then 5min diagram was recorded, and aggregation rates at 1min, 3min and 5min and the maximum aggregation rates were read.

### 3.2 Experiments of artery-vein bypass thrombosis

Rats were anesthetized by intraperitoneal injection of 2% pentobarbital sodium solution (30-40mg/kg), fixed at supine position, right common carotid artery and left external jugular vein were separated, three sections of polyethylene pipes were connected, one end thereof was inserted in the right common carotid artery and the other end thereof was inserted in the left external jugular vein, the polyethylene pipe at the two ends were filled with 25u/ml heparin (freshly prepared with physiological saline solution), and the middle section had a length of 10cm, a 7^{#} suture (weighed) with a length of 8cm was placed in the pipe which was filled with physiological saline solution (notice: no bubble was allowed), so that an artery-vein bypass was established. The pipe was taken down after 15min, thrombus was taken out, rolled on a wet filter paper, residual blood was removed, placed on a parchment paper and weighed to obtained its wet weight. Then, it was placed in a drying oven and dried at 60°C for 1 h to reach a constant weight, cooled and weighed to obtain the dry weight of thrombus.

### 3.3 Test of caudal vein bleeding time in mice

Mice were randomly divided into 7 groups: control group, aspirin group (ASP,40mg/Kg), BII group (40 mg/Kg), AIII group (40 mg/Kg), 1:1 group (weight ratio of AIII to BII, 40 mg/Kg), 1:3 group (weight ratio of AIII to BII, 40 mg/Kg), 3:1 group (weight ratio of AIII to BII, 40 mg/Kg). Continuous gastric administration was performed for 5 days, twice per day, and the experiments were conducted after 1h of the administration on the 6^{th} day. The control group was administered with physiological saline of the same volume. All drug ratios were AIII to BII.

Mice were anesthetized with pentobarbital sodium (40-60mg/kg), placed on a worm and comfortable pad, a tail tip was cut at a place where the tail of mice had a diameter of 2.25-2.5mm, and immediately placed in PBS (37°C), timing started. Hemostatic time and re-bleeding time within 10min were observed, and the bleeding volume of mice was presumed by hemoglobin volume. If bleeding did not stop within 10min, hemostasis was performed by compression, and the hemostatic time was recorded as 600 seconds.

### 3.4 Lethal time in mice by caudal vein injection of collagen

Mice were randomly divided into 7 groups: control group, aspirin group (ASP,40mg/Kg), BII group (40 mg/Kg), AIII group (40 mg/Kg), 1:1 group (weight ratio of AIII to BII, 40 mg/Kg), 1:3 group (weight ratio of AIII to BII, 40 mg/Kg), 3:1 group (weight ratio of AIII to BII, 40 mg/Kg). Continuous gastric administration was performed for 5 days, twice per day, and the experiments were conducted after 1 h of the administration on the 6^{th} day. The control group was administered with physiological saline of the same volume.

Mice were fixed on mice-fixed frame, and thrombotic agent (100µg/mouse) consisting of collagen (0.5mg/kg) and adrenalin (60ug/kg) were administered by caudal vein injection. The symptoms, symptom occurrence time, and the lethal time in mice were observed within 10min, and mice dead after 10min were deemed as survival.

### II. Experimental results

### 1. Effects of timosaponins via gastric administration on platelet aggregation rate in mice

Blood samples were collected from heart of anaesthetized mice after 1 h of the last gastric administration of timosaponins, and analyzed to determine the rates of platelet aggregation induced by ADP (20µM) after administration. The results showed that, in comparison with the control group, the maximum aggregation rates of the AIII group, 3:1 group and 1:1 group decreased significantly, in which the platelet aggregation inhibitive effects of the AIII group and 3:1 group were equivalent to that of aspirin group. This indicates that the platelet aggregation inhibitive effect of timosaponin AIII was greater than that of BII, but the 3:1 group still had significant activity of combating platelet aggregation.

**Table 1: Effects of the pharmaceutical composition of the present invention on thrombosis by administration in vivo**

| Group | number | Maximum aggregation rate (%) |
|---|---|---|
| Control group | 6 | 54.9±5.8 |
| ASP 40 mg/Kg group | 6 | 40.8±7.6** |
| AIII 40 mg/Kg group | 6 | 39.8±5.0** |
| BII 40 mg/Kg group | 6 | 50.8±6.7 |
| 1:1 40 mg/Kg group | 6 | 44.5±9.4* |
| 1:3 40 mg/Kg group | 6 | 48.3±5.6 |
| 3:1 40 mg/Kg group | 6 | 42.2±4.5** |

| | | |
|---|---|---|
| Note: in comparison with the control group: **P< 0.01, *P< 0.05 | | |

### 2. Experiments of artery-vein bypass thrombosis

In comparison with normal control, the thrombus wet weight and dry weight of all administration groups exhibited a declining tendency, in which the thrombus wet weights of the AIII group and 3:1 group were significantly different from that of the control group, the 1:1 group had difference, but the BII group and 1:3 group showed no statistic difference in comparison with the control group. It can be seen from the experimental results that AIII played a leading role in antithrombotic aspect. The thrombus wet weight of the AIII group was less than that of the positive control drug, aspirin, which indicates that AIII is better than aspirin in antithrombotic aspect. The specific experimental results are shown in Table 2.

**Table 2: Effects of the pharmaceutical composition of the present invention on thrombosis by administration in vivo**

| Group | number | Thrombus wet weight (mg) | Thrombus dry weight (mg) |
|---|---|---|---|
| Control group | 11 | 117.12±38.33 | 35.9±8.35 |
| ASP 40 mg/Kg group | 11 | 79.74±18.95** | 25.5±7.37** |
| AIII 40 mg/Kg group | 8 | 64.29±22.58** | 24.4±6.30** |
| BII 40 mg/Kg group | 9 | 90.39±29.59 | 28.6±7.82 |
| 1:1 40 mg/Kg group | 8 | 87.75±14.24* | 29.6±6.52 |
| 3:1 40 mg/Kg group | 7 | 70.31±12.73** | 26.6±8.10* |
| 1:3 40 mg/Kg group | 6 | 88.60±17.17 | 29.5±5.35 |

| | | | |
|---|---|---|---|
| Note: in comparison with the control group: **P< 0.01, *P< 0.05 | | | |

### 3.3 Experiments of the lethal time by caudal vein injection of collagen in mice

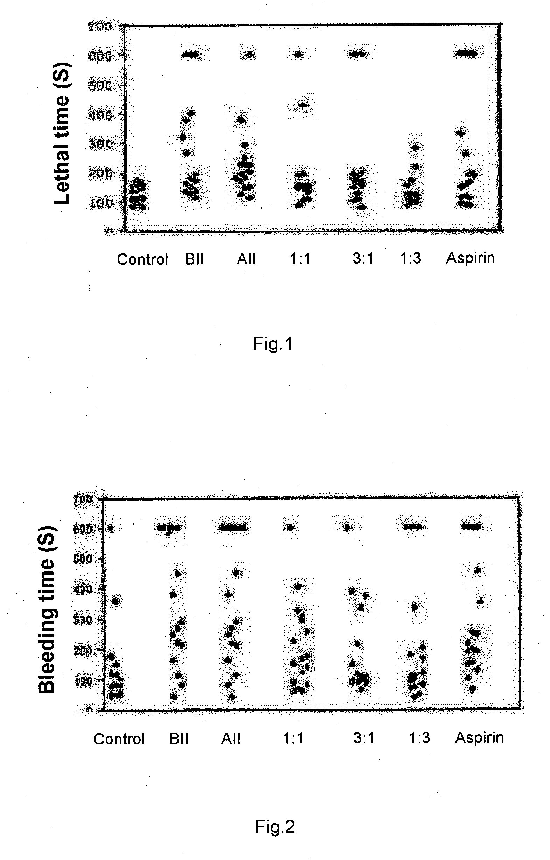

After caudal vein injection of thrombotic agent, mice appeared symptoms such as breathing rapidly, restless, rotation and ocular proptosis, and then dead quickly. The average lethal time of the control group was 129.3±26.9 s, while the lethal time of the animals administered with BII and AIII singly were significantly extended, and were 259.1±169.9 s and 237.9±125.1 s, respectively, which were significantly different from that of the control group. The lethal time of the animals administered with the combinations of the two drugs was also extended in some extent in comparison with the control group, but except for the 3:1 group, their effects were inferior to that of the groups administered with single drugs. The lethal time and survival rate of the 3:1 group were similar to those of the groups administered with single drugs, which indicated that AIII played a leading role in the combination of drugs. The specific survival rates and the lethal time of the groups are shown in Table 3 and Fig.1.

**Table 3: Effects of the pharmaceutical composition administrated in vivo on the lethal time by caudal vein injection of collagen in mice**

| Group | number | Lethal time (s) |
|---|---|---|
| Control group | 15 | 129.3±26.9 |
| ASP 40 mg/Kg group | 15 | 244.6±195.3* |
| AIII 40 mg/Kg group | 14 | 237.9±125.1** |
| BII 40 mg/Kg group | 14 | 259.1±169.9** |
| 1:1 40 mg/Kg group | 13 | 198.6±140.2 |
| 3:1 40 mg/Kg group | 13 | 254.6±199.5* |
| 1:3 40 mg/Kg group | 12 | 142.7±56.9 |

| | | |
|---|---|---|
| Note: in comparison with the control group: ** P< 0.01, * P< 0.05 | | |

### 3.2 Experiments of caudal vein bleeding time in mice

The average hemostatic time of the control group was 88.9±45.9 s, the bleeding time of the administration groups were significantly extended in comparison with the control group, the aspirin group as positive control had an average hemostatic time of 277.4±188.5 s, which was extremely significantly different from the normal control group. The average hemostatic time when BII and AIII were solely administered were greater than that of the positive control, which indicated the administration of single timosaponin BII or AIII had significant bleeding tendency. In comparison with the control group, the bleeding time of the combination of drugs also extended in some extent, but the bleeding volume was significantly less those of the groups administered with single drugs and the aspirin group. This indicated that although timosaponins BII and AIII had potent antithrombotic effect, they also had significant bleeding tendency; the reasonable combination of them (the 3:1 group) had antithrombotic activity and significantly less bleeding time and total bleeding volume. The specific results are shown in Table 4 and Fig.2.

**Table 4: Effects of the pharmaceutical composition administrated in vivo on caudal vein bleeding time and bleeding volume in mice**

| Group | number | Hemostatic time (s) | Bleeding volume (µL) |
|---|---|---|---|
| Control group | 17 | 88.9±45.9 | 11.3±8.3 |
| ASP 40 mg/Kg group | 17 | 277.4±188.5*** | 13.5±13.8 |
| AIII 40 mg/Kg group | 17 | 396.6±208.9 *** | 21.3±29.6 |
| BII 40 mg/Kg group | 17 | 325.1±200.6*** | 10.7±9.8 |
| 1:1 40 mg/Kg group | 17 | 209.9±148.0**§§ | 6.0±5.2 §§ |
| 3:1 40 mg/Kg group | 16 | 200.3±162.0*#§§ | 5.5±6.5#§§ |
| 1:3 40 mg/Kg group | 17 | 209.7±206.7*§§ | 11.6±13.7§§ |

| | | | |
|---|---|---|---|
| Note: in comparison with the control group: P< 0.001, P< 0.01, P< 0.05 In comparison with the BII group: ### P< 0.001; ## P< 0.01; # P< 0.05 In comparison with the AIII group: §§§P< 0.001; §§P< 0.01; §P< 0.05 | | | |

### III. Discussions

It was found in the in vitro activity screening that timosaponin AIII had significant inhibitive effect on platelet aggregation, while timosaponin BII had significant effect of expanding blood vessels. The results of animal in vivo experiments that timosaponin AIII had the strongest activity of inhibiting in vivo platelet aggregation and combating artery-vein bypass thrombosis in rats, BII had a weaker activity, and different combinations of timosaponins, especially the 3:1 combination of timosaponin AIII and BII, also had activity of inhibiting platelet aggregation and combating thrombosis.

In the lethal experiments with caudal vein injection of collagen, both timosaponin AIII with function of combating platelet aggregation and timosaponin BII with function of expanding blood vessels could significantly extend the survival time of the model mice and increase the survival rate of the model mice, but the mice tail cutting experiments showed that the caudal vein bleeding time were extended and bleeding volume increased when mice were singly administered with these two compounds. In the meantime, it was also found that the 3:1 combination of timosaponins AIII and BII also had activity of combating thrombosis that was equivalent to those of administering them singly, although the caudal vein bleeding time was extended, the bleeding volume decreased significantly; although the caudal vein bleeding time and bleeding volume of other combination of timosaponins decreased, their antithrombotic activity decreased significantly as well. The experiments showed that a relevantly strong activity of inhibiting platelet aggregation and a suitable activity of expanding blood vessels could bring about the best antithrombotic effect in vivo.

Hence, one of the important findings in the experiments is that the combinations of timosaponins with a major proportion of AIII can bring about the best antithrombotic effect in vivo, and have relatively less bleeding side-effects. Hence, medicaments of various dosage forms have been developed, can be used for treatment of various kinds of cerebrovascular disease and fulfill the purpose of safe and high efficiency.

### Example 2: Study of the in vitro inhibitive effect of the present pharmaceutical composition on platelet aggregation

### I. Materials and Methods

### 1. Experimental materials

Wistar rats, male, body weight 280-320 g, big-ear rabbits, female, body weight 2-2.5kg, macaques, male, body weight 5-7kg, all provided by the Animal Breeding Center of the Academy of Military Medical Sciences. Human platelet was provided by Beijing Blood Center. The present pharmaceutical composition was a white powder prepared according to the method recited in Example 15. Adenosine diphosphate (ADP), arachidonic acid (AA), dimethyl sulfoxide (DMSO) were products of Sigma Company; Ristocetin and adrenalin were purchased from Biopool Company.

### 2. Experimental methods

### 2.1 Collecting blood

Male Wistar rats were anesthetized by intraperitoneal injection of 2% pentobarbital sodium (30-40 mg/kg), and blood samples were collected from heart; blood samples were collected from ear arteries of female bid-ear rabbits at quiet state, and then immediately anticoagulated with 3.8% sodium citrate (volume ratio: 1:9).

### 2.2 Preparation of platelet

The collected blood samples were centrifuged under 800rpm and room temperature for 10min, the upper layer of platelet rich plasma was taken, centrifuged under 3000rpm and room temperature for 20min, the upper layer of platelet poor plasma were taken. Counting was performed by using F-820 blood cell counter, and PRP was adjusted with PPP to have a concentration of 3.0×10¹¹/L.

### 2.3 Determination of maximum platelet aggregation rate

Chronolog platelet aggregation analyzer test: Platelet suspension was adjusted with plasma to have a concentration of 3.0×10¹¹/L, the platelet aggregation analyzer was turned on, and PPP was used as a blank control to adjust transmittance to be 100%. 450µL of platelet suspension were taken, stirred with a stir bar and pre-warmed at 37°C for 3min, and separately added with various kinds of inducing agents (50µL): ADP agent (final concentration: 20 µM), AA (final concentration: 80µM), Ristocetin (final concentration: 1.2mg/mL), adrenalin (final concentration: 10µM), then 5min diagram was recorded, and aggregation rates at 1 min, 3min and 5min and the maximum aggregation rates were read.

### II. Experimental results

### 1. Effects of the present pharmaceutical composition on in vitro inhibiting platelet aggregation induced by ADP in rats

Platelet samples of rats were pre-added with the present pharmaceutical composition at different concentrations, incubated for 2min, added with ADP as aggregation inducer, and the platelet aggregation rates at different time were detected. The results were shown in Table 5, in which 10 µg/mL of the present pharmaceutical composition could significantly inhibit the aggregation of rat platelets, and the inhibitive effect increased significantly with the increase of the dose of the present pharmaceutical composition, and the present pharmaceutical composition at 50µg/mL level can completely inhibit the aggregation of rat platelets. It was calculated by statistic treatment that the IC₅₀ value of the present pharmaceutical composition for inhibiting rat platelet aggregation was 26.92±4.75 µg/mL.

**Table 5: Inhibitive effects of the pharmaceutical composition of the present invention on platelet aggregation in rats (n=8)**

| Group | Platelet aggregation rate (%) | | | |
|---|---|---|---|---|
| | 1min | 3min | 5min | Max. |
| ADP 20 µM | 22.4±2.83 | 39.0±1.77 | 43.9±2.42 | 43.9±2.42 |
| ADP 20 µM + the present pharmaceutical composition 10 µg/mL | 19.6±5.32 | 35.6±3.50 | 37.5±3.82 | 38.13±3.04** |
| ADP 20 µM + the present pharmaceutical composition 20 µg/mL | 16.0±4.44 | 30.6±5.32 | 27.5±8.75 | 31.38±6.74** |
| ADP 20 µM + the present pharmaceutical composition 30 µg/mL | 9.8±5.78 | 19.0±9.47 | 14.4±12.32 | 20.75±8.78** |
| ADP 20 µM + the present pharmaceutical composition 40 µg/mL | 7.3±5.50 | 14.0±10.28 | 10.8±8.63 | 15.25±8.83** |
| ADP 20 µM + the present pharmaceutical composition 50 µg/mL | 0.4±0.74 | 0.1±0.93 | 0 | 1.0±1.85** |

| | | | | |
|---|---|---|---|---|
| Note: comparing the maxium aggregation rate to the ADP group: *P<0.05, **P<0.01 | | | | |

### 2. Inhibitive effect of the present pharmaceutical composition on platelet aggregation in vitro in rabbits

Rabbits are one of common animals for studying antithrombotic drugs. In the experiments, the inhibitive effects of the present pharmaceutical composition in different doses on rabbit platelet aggregation were observed. The results were shown in Table 6, in which 10 µg/mL of the present pharmaceutical composition can inhibit the maximum rate of ADP-induced rabbit platelet aggregation by 23%, the inhibitive effect increased significantly with the increase of the dose of the present pharmaceutical composition, and the present pharmaceutical composition at 60 µg/mL level could inhibit the maximum rate of rabbit platelet aggregation by 80%. It was calculated by statistic treatment that the IC₅₀ value of the present pharmaceutical composition for inhibiting rabbit platelet aggregation was 16.1± 2.1 µg/mL.

**Table 6: Inhibitive effects of the pharmaceutical composition of the present invention on platelet aggregation in rabbits (n=3)**

| Group | Maximum aggregation rate (%) | | | |
|---|---|---|---|---|
| | 1min | 3min | 5min | Max. |
| ADP 20 µM | 16.7±2.08 | 32.0±2.65 | 35.7±4.73 | 35.7±4.73 |
| ADP 20 µM + the present pharmaceutical composition 10 µg/mL | 18.0±5.20 | 26.5±4.50 | 26.7±3.79 | 27.3±3.51** |
| ADP 20 µM + the present pharmaceutical composition 20 µg/mL | 10.5±4.09 | 14.7±3.51 | 14.2±3.75 | 15.2±3.33** |
| ADP 20 µM + the present pharmaceutical composition 30 µg/mL | 9.0±4.24 | 10.4±4.74 | 9.5±0.36 | 11.0±4.24** |
| ADP 20 µM + the present pharmaceutical composition 40 µg/mL | 7.3±1.77 | 7.5±2.12 | 7.0±4.24 | 9.0±1.41** |
| ADP 20 µM + the present pharmaceutical composition 60 µg/mL | 0.4±0. 74 | 5.0±1.41 | 3.8±3.89 | 6.8±0.35** |

| | | | | |
|---|---|---|---|---|
| Note: comparing the maximum aggregation rate to the ADP group: *P<0.05, **P<0.01 | | | | |

### 3. Inhibitive effects of the present pharmaceutical composition on macaque platelet aggregation in vitro

Macaques have the closest genetic background to human. The inhibitive effects of the present pharmaceutical composition on macaque platelet aggregation were observed. It was found in the experiment that the present pharmaceutical composition at 50 µg/mL could inhibit the maximum rate of the ADP-induced macaque platelet aggregation by 13%, the inhibitive effects increased with the increase of the dose of the present pharmaceutical composition, and the present pharmaceutical composition at 150 µg/mL could inhibit the maximum rate of macaque platelet aggregation by 90%. It was calculated by statistic treatment that the IC₅₀ value of the present pharmaceutical composition for inhibiting macaque platelet aggregation was 79.16±5.31 µg/mL. it can be seen that the IC₅₀ value of the present pharmaceutical composition for inhibiting macaque platelet aggregation was significantly higher than those for rabbits and rats.

**Table 7: Inhibitive effects of the pharmaceutical composition of the present invention on platelet aggregation in macaques (n=5)**

| Group | Maximum aggregation rate (%) | | | |
|---|---|---|---|---|
| | 1min | 3min | 5min | Max. |
| ADP 20 µM | 34.8±1.94 | 54±5.02 | 58.2±5.84 | 58.2±5.84 |
| ADP 20 µM + the present pharmaceutical composition 50 µg/mL | 24.6±1.74 | 40.4±2.24 | 45±3.46 | 45±3.46** |
| ADP 20 µM + the present pharmaceutical composition 75 µg/mL | 15±4.20 | 29.4±5.35 | 32.8±5.31 | 32.8±5.31** |
| ADP 20 µM + the present pharmaceutical composition 100 µg/mL | 11.6±4.41 | 20.6±4.76 | 23.6±3.72 | 23.6±3.72** |
| ADP 20 µM +the present pharmaceutical composition 125 µg/mL | 4.5±1.12 | 9.8±1.79 | 12.5±2.06 | 12.5±2.06** |
| ADP 20 µM + the present pharmaceutical composition 150 µg/mL | 0 | 4±2.94 | 7.3±2.36 | 7.3±2.36** |

| | | | | |
|---|---|---|---|---|
| Note: comparing the maximum aggregation rate to the ADP group: *P<0.05, **P<0.01 | | | | |

### 4. Comparison of in vitro inhibitive effects of the present pharmaceutical composition on human platelet aggregation induced by different inducing agents

Fresh platelets from health human were added with the present pharmaceutical composition of different concentrations, then platelet aggregation was induced with various kinds of inducing agent, and the results are shown in Tables 8-11. It was found in the experiments that ADP (20 µM), arachidonic acid (80 µM), Ristocetin (1.2 mg/mL) and adrenalin (10 µM) all induced human platelet aggregation, and aggregation rates were all above 50%. The present pharmaceutical composition at 20-25 µg/mL could inhibit the platelet aggregation induced by all of the above inducing agents, the inhibitive effects increased with the increase of the dose of the present pharmaceutical composition, and the present pharmaceutical composition at 150-300 µg/mL could completely inhibit the human platelet aggregation induced by the above inducing agents. The IC₅₀ value of the present pharmaceutical composition for inhibiting human platelet aggregation induced by different inducing agents were shown in Table 12.

The above experiment showed that platelets from different sources had difference in sensibility to the present pharmaceutical composition, while the pharmaceutical composition at a large dose level could completely inhibit the human platelet aggregation induced by ADP, arachidonic acid, Ristocetin and adrenalin, which suggested the present pharmaceutical composition had potential clinical application value.

**Table 8: Effects of the present pharmaceutical composition on human platelet aggregation induced by ADP (n=5)**

| Group | Maximum aggregation rate (%) | | | |
|---|---|---|---|---|
| | 1min | 2min | 3min | 5min |
| ADP 20 µM | 23.5±3.87 | 34.50±1.73 | 42.75±2.75 | 51.25±9.07 |
| ADP 20 µM + the present pharmaceutical composition 12.5 µg/mL | 23.25±2.2 2 | 33.00±2.16 | 42.25±5.91 | 50.75±9.11 |
| ADP 20 µM + the present pharmaceutical composition 25 µg/mL | 23.75±4.9 2 | 32.25±4.11 | 41.75±6.55 | 46.75±9.84** |
| ADP 20 µM + the present pharmaceutical composition 50 µg/mL | 21.00±2.5 8 | 30.00±4.55 | 36.75±6.90 | 40.75±8.42** |
| ADP 20 µM + the present pharmaceutical composition 100 µg/mL | 10.50±1.7 3 | 17.00±0.82 | 20.50±1.29 | 24.00±4.32** |
| ADP 20 µM + the present pharmaceutical composition 200 µg/mL | 0.25±0.50 | 0.50±1.00 | 0.75±1.50 | 1.50±1.91** |

| | | | | |
|---|---|---|---|---|
| Note: comparing the maximum aggregation rate to the ADP group, *P<0.05, **P<0.01 | | | | |

**Table 9: Effects of the present pharmaceutical composition on human platelet aggregation induced by AA (n=5)**

| Group | Maximum aggregation rate (%) | | | |
|---|---|---|---|---|
| | 1min | 2min | 3min | 5min |
| AA 80 µM | 39.00±9.50 | 53.03±7.30 | 62.00±5.50 | 67.75±2.25 |
| AA 80 µM + the present pharmaceutical composition 25 µg/mL | 18.00±3.50 | 38.13±2.83 | 49.50±10.50 | 63.25±3.75 |
| AA 80 µM + the present pharmaceutical composition 50 µg/mL | 16.25±4.75 | 42.25±3.75 | 54.75±2.96 | 61.25±3.25* |
| AA 80 µM + the present pharmaceutical composition 100 µg/mL | 11.00±7.00 | 29.15±6.24 | 37.75±3.38 | 43.75±3.25** |
| AA 80 µM + the present pharmaceutical composition 200 µg/mL | 7.50±1.25 | 18.27±1.85 | 28.75±10.63 | 33.00±9.50** |
| AA 80 µM + the present pharmaceutical composition 300 µg/mL | 0 | 0 | 0 | 0** |

| | | | | |
|---|---|---|---|---|
| Note: comparing the 5min maximum aggregation rate to the AA group: *P<0.05, **P<0.01 | | | | |

**Table 10: Effects of the present pharmaceutical composition on human platelet aggregation induced by Ristocetin (n=5)**

| Group | Maximum aggregation rate (%) | | | |
|---|---|---|---|---|
| | 1min | 2min | 3min | 5min |
| Ristocetin 1.2 mg/mL | 38.67±4.62 | 54.33±6.35 | 60.67±5.13 | 64.67±4.04 |
| Ristocetin 1.2 mg/mL + the present pharmaceutical composition 25 µg/mL | 32.33±2.52 | 51.67±5.86 | 57.67±6.66 | 63.00±3.61 |
| Ristocetin 1.2 mg/mL + the present pharmaceutical composition 50 µg/mL | 30.67±6.43 | 49.33±7.23 | 53.00±7.00 | 58.33±2.89* |
| Ristocetin 1.2 mg/mL + the present pharmaceutical composition 100 µg/mL | 23.33±1.15 | 43.00±6.08 | 49.00±7.21 | 54.00±2.65* |
| Ristocetin 1.2m g/mL + the present pharmaceutical composition 200 µg/mL | 2.00±2.00 | 6.67±3.06 | 12.33±1.15 | 24.67±4.16** |
| Ristocetin 1.2 mg/mL + the present pharmaceutical composition 400 µg/mL | 0 | 0 | 0.33±0.58 | 0.33±0.58** |

| | | | | |
|---|---|---|---|---|
| Note: comparing the maximum aggregation rate to the Ristocetin group: *P<0.05, **P<0.01 | | | | |

**Table 11: Effects of the present pharmaceutical composition on human platelet aggregation induced by adrenalin (n=5)**

| Group | Maximum aggregation rate (%) | | | |
|---|---|---|---|---|
| | 1min | 2min | 3min | 5min |
| Adrenalin 10 µM | 13.67±3.87 | 44.33±1.53 | 50.67±1.53 | 55.67±1.15 |
| Adrenalin + the present pharmaceutical composition 25 µg/mL | 17.33±2.08 | 41.33±9.07 | 49.33±5.13 | 55.33±0.58 |
| Adrenalin + the present pharmaceutical composition 50 µg/mL | 9.67±2.52 | 21.33±3.51 | 35.00±5.57 | 43.33±4.04* |
| Adrenalin + the present pharmaceutical composition 75 µg/mL | 9.67±2.52 | 19.00±1.73 | 31.67±3.51 | 35.33±1.15** |
| Adrenalin + the present pharmaceutical composition 100 µg/mL | 1.33±0.58 | 2.67±0.58 | 3.33±0.58 | 4.33±2.08** |
| Adrenalin + the present pharmaceutical composition 150 µg/mL | 0.33±0.58 | 0.33±0.58 | 0.67±0.58 | 1.33±0.58** |

| | | | | |
|---|---|---|---|---|
| Note: comparing the maximum aggregation rate to the adrenalin group: *P<0.05, **P<0.01 | | | | |

**Table 12: The IC₅₀ values of the present pharmaceutical composition on human platelet aggregation induced by different inducing agents**

| | ADP | Arachidonic acid | Ristocetin | Adrenalin |
|---|---|---|---|---|
| IC50 (µg/mL) | 81.28±3.64 | 131.92±16.75 | 147.54±9.34 | 89.42±5.74 |

### Example 3: Study of inhibitive effects of the in vivo administration of the present pharmaceutical composition on thrombosis

### I. Materials and Methods

### 1. Experimental materials

Wistar rats, male, body weight 280 ∼ 320g, were provided by the Animal Breeding Center of the Academy of Military Medical Sciences. The composition of timosaponins BII and AIII was obtained by the preparation method recited in Example 5. Heparin was purchased from Sigma Company. Physiological saline solution was purchased from Beijing Shuanghe Pharmaceutical Group Ltd. Aspirin was from Ouyi Pharmaceutical Company of Shijiazhuang Pharmaceutical Group Ltd. Kits for bleeding time and coagulation time (PT, TT and APTT) were purchased from Shanghai Taiyang Biotechnology Company.

### 2. Experimental methods

### 2.1 Grouping animals

Wistar rats were randomly divided into 5 groups, i.e., blank control group, aspirin 40 mg/kg group, the present pharmaceutical composition 10 mg/kg, 20 mg/kg and 40 mg/kg groups, the animals of the groups were subjected to continuous gastric administration for 7 days, once per day, dose volume was 10 mL/kg, operation was conducted after 1h of administration on the 7^{th} day, and the blank control was administered with distilled water of the same volume.

### 2.2 Experiments of artery-vein bypass thrombosis in rats

The method of reference document was slightly improved. Rats were anesthetized by intraperitoneal injection of 2% pentobarbital sodium solution (30-40mg/kg), fixed at supine position, right common carotid artery and left external jugular vein were isolated, three sections of polyethylene pipes were connected, one end thereof was inserted in the right common carotid artery and the other end thereof was inserted in the left external jugular vein, the polyethylene pipe at the two ends were filled with 25u/ml heparin (freshly prepared with physiological saline solution), and the middle section had a length of 10cm, a 7# suture (weighed) with a length of 8cm was placed in the pipe which was filled with physiological saline solution (notice: no bubble was allowed), so that an artery-vein bypass was established. The pipe was taken down after 20min, thrombus was taken out, rolled on a wet filter paper, residual blood was removed, placed on a parchment paper and weighed to obtained its wet weight. Then, it was placed in a drying oven and dried at 60°C for 1 h to reach a constant weight, cooled and weighed to obtain the dry weight of thrombus.

### 2.3 Preparation of PRP and PPP

Wistar male rats, after 1h of administration on the 7^{th} day, were anesthetized by intraperitoneal injection of pentobarbital sodium (30-40 mg/kg), blood samples were collected from hearts, anti-coagulated with 3.8% sodium citrate (volume ratio: 1:9). Centrifugation was performed under 800rpm and room temperature for 10min, the upper layer of platelet-rich plasma (PRP) was taken, centrifuged under 3000rpm and room temperature for 20min, the upper layer of platelet-poor plasmas (PPP) was taken. Counting was performed by using F-820 blood cell counter, and PRP was adjusted with PPP to have a concentration of 3.0×10¹¹/L.

### 2.4 Determination of maximum platelet aggregation rate

Chronolog platelet aggregation analyzer test: Platelet suspension was adjusted with plasma to have a concentration of 3.0×10¹¹/L, the platelet aggregation analyzer was turned on, and PPP was used as a blank control to adjust transmittance to be 100%. 450µL of platelet suspension were taken, stirred with a stir bar and pre-warmed at 37°C for 3min, and separately added with inducing agent (50µL) ADP agent (final concentration: 20 µM), then 5min diagram was recorded, and aggregation rates at 1m in, 3m in and 5min and the maximum aggregation rates were read.

### 2.5 Determination of bleeding time and coagulation time

Blood samples were collected, plasmas were separated, and the operation was conducted according to the specification of kits.

### II. Experimental results

### 1. Effects of the gastric administration of the present pharmaceutical composition on rat artery-vein bypass thrombosis

The present pharmaceutical composition was used for gastric administration, the doses for the administration were 40, 20 and 10 mg/Kg, respectively, once per day, aspirin 40 mg/Kg was the positive control, the artery-vein bypass thrombosis experiments were conducted on the 7^{th} day after the administration. The results showed that the groups of large dose and middle dose of the present pharmaceutical composition gave significantly decreased thrombus dry weight and wet weight in comparison with the control group, in which the decrease in the large dose group was the most significant, and the small dose group gave a thrombus weight less than that of the control group but had no significant difference. The aspirin positive control group gave thrombus dry and wet weights slightly higher than those of the large dose group, and had significant difference from the control group. These results suggested that the present pharmaceutical composition had effect of inhibiting thrombosis in vivo.

**Table 13: Effects of the present pharmaceutical composition by gastric administration on jugular artery-vein bypass thrombosis in rats**

| Group | number | Thrombus weight (mg) | |
|---|---|---|---|
| | | Wet weight | Dry weight |
| Control group | 11 | 117.1±38.3 | 35.9±8.4 |
| the present pharmaceutical composition 40 ug/mL | 9 | 64.3±22.6** | 24.4±6.3** |
| the present pharmaceutical composition 20 ug/mL | 8 | 70.3±12.7* | 26.6±8.1* |
| the present pharmaceutical composition 10 ug/mL | 8 | 88.6±17.2 | 29.5±5.4 |
| Aspirin group | 11 | 79.7±19.0* | 26.7±6.4* |

| | | | |
|---|---|---|---|
| Note: in comparison with the control group: **p<0.01; *p<0.05 | | | |

### 2. Effects of the present pharmaceutical composition by gastric administration on platelet aggregation rate and bleeding/coagulation time in rats

The administration method of the present pharmaceutical composition and the grouping method were identical to that of the third part, blood samples were collected from hearts of rats on the 7^{th} day after administration, and the ADP-induced platelet aggregation and bleeding/coagulation time (PT, TT and APTT) of the same batch were determined. The results showed that the 3 dose groups of the present pharmaceutical composition all gave platelet aggregation rates lower than that of the control group, and had a certain dose effect. The aspirin group gave a platelet aggregation rate lower than that of the control group, but had no significant difference. The groups treated with the present pharmaceutical composition did not have statistic difference in comparison with the control group in aspects of PT, TT and APTT; the aspirin group gave a significantly extended APTT in comparison with the control group, but had no significant difference in comparison with the control group in other two indexes. The above results showed that the in vivo administration of the present pharmaceutical composition could inhibit platelet aggregation, provent thrombosis, but did not influence blood coagulation time.

**Table 14: Effects of the present pharmaceutical composition by gastric administration on ADP-induced platelet aggregation rate in rats**

| | Control group | Composition of AIII and BII, 40 ug/mL | Composition of Allland BII, 20 ug/mL | The present pharmaceutical composition, 10 ug/mL | Aspirin group |
|---|---|---|---|---|---|
| PLT maximum aggregation rate | 54.9±5.8 | 43.3±7.6** | 48.3±5.1* | 49.9±4.6 | 49.1±4.8 |

| | | | | | |
|---|---|---|---|---|---|
| Note: in comparison with the control group: **p<0.01; *p<0.05 | | | | | |

**Table 15: Effects of the present pharmaceutical composition by oral administration on rat blood coagulation parameters APTT, PT and TT**

| Group | n | Coagulation time (s) | | |
|---|---|---|---|---|
| | | APTT | PT | TT |
| Normal control group | | | | |
| the present pharmaceutical composition 40 mg/kg | 8 | 24.84±1.45 | 17.74±1.55 | 28.37±0.90 |
| | 8 | 24.91±1.46 | 16.85±1.11 | 27.34±1.58 |
| the present pharmaceutical composition 20 mg/kg | 8 | 24.53±1.07 | 17.15±1.15 | 28.20±1.84 |
| | 8 | 24.47±1.67 | 16.91±0.76 | 27.65±2.32 |
| the present pharmaceutical composition 10 mg/kg aspirin group | 8 | 17.50±2.37* | 16.49±2.08 | 28.98±0.97 |

| | | | | |
|---|---|---|---|---|
| Note: in comparison with the normal control group: *P<0.05 | | | | |

### Example 4: Effects of the present pharmaceutical composition on motion and sensory function in cerebral ischemia rats

The best therapeutic time window for ischemic cerebrovascular diseases (mainly referring to cerebral thrombosis) is within 6h after onset, and positive therapy may reduce injury to the lowest limit. However, most cerebral ischemia patients are attacked at rest state such as sleeping state, and transportation and imaging diagnosis often cause the delay of treatment and the residue of sequel such as hemiplegia and aphasia. Hence, the treatment and intervention during cerebral subacute phase and early recovery phase are especially important. In the experiment, a middle cerebral artery occlusion (MCAO) model was used for studying the effects of the present pharmaceutical composition on motion function of cerebraon ischemic injury rats, and thus an experimental basis was provided for reasonable application of the present pharmaceutical composition in clinic.

### I. Materials and Methods

### 1. Experimental animals

SD rats, male, body weight 280-300g, were provided by Beijing Weitonglihua Experimental Animals Technology Co., Ltd., Certificate No.: SCXK ( 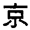 ) 2002-2003.

### 2. Experimental agents

The present pharmaceutical composition was prepared according to the method of Example 6.

### 3. Experimental methods

### 3.1 Preparation of rat middle cerebral artery occlusion reperfusion model

Rats were anesthesized by intraperitoneal injection of 10% chloral hydrate, supinely fixed, conventionally sterilized skin, subjected to anterior neck cut, separation to expose right common carotid artery, internal carotid artery and external carotid artery, passed by a suture for standby use, the external carotid artery and the common carotid artery were ligatured, the distal end of the internal carotid artery was clipped with an artery clip, an incision was cut on the bifurcation of the external carotid artery and the internal carotid, and a smooth nylon suture (diameter 0.25mm, end diameter 0.27mm, marked at a place 18mm from the end) with an end ground as ball shape was inserted into the incision, and stopped when resistance was felt, the ischemic time was recorded, the insertion depth was about 18mm to achieve cerebral ischemia caused by middle cerebral artery occlusion. The incision was then ligatured, the nylon suture was fixed, muscle and skin were sutured layer by layer, and sterilized. After 2h, the nylon suture was pulled until its end was close to the incision to fulfill reperfusion. The rats of the sham group were merely subjected to explosion and separation of right common carotid artery. The room temperature during the cerebraon ischemia and reperfusion was kept at 23°C, and the rats were raised in cages by conventional method.

### 3.2 Judgement of success of model

According to Zea Longa 5 grade scoring method (please give its references), the rats were scored after complete sober, the scoring criterion was: no apparent neurological symptom, scoring 0; not capable of completely stretching left front paw, scoring 1; turning left side, scoring 2; falling on left side during walking, scoring 3; not capable of walking by itself, scoring 4. The rats scored 1-3 were selected for the sequent tests.

### 3.3 Grouping and administration

The rats whose nervous symptoms were scored 1-3 were divided into 5 groups: model group; the groups administered with the present pharmaceutical composition 15 mg/kg (low), 30 mg/kg (middle), 60 mg/kg (high); Angong Niuhuang Wan (please give its manufacturer and batch number) 400 mg/kg was used as positive control. The sham group and the model group were administered with equivalent amount of 0.5 % CMC solution. From the 3^{rd} to 14^{th} day after operation, the rats were gastrically administered, once per day.

### 3.4 Detection of limb motion and sensory function

### 3.4.1 Beam walking test

Beam walking test [Feeney DM et al, Science, 1982, 217: 855-857] was used for evaluating the coordination and integration deficiency of motion. A beam with a width of 2.0cm, a length of 120cm and a thickness of 1 cm was honzontally suspended in midair 80cm above the ground, one end of the beam was connected to a dark box (length 25cm, width 22cm, height 18cm), and the rats were stimulated with noise to pass the beam and enter the dark box. Scoring criterion: rat is unable to stay on the beam, scoring 0; rat is able to stay on the beam but unable to move, scoring 1; rat tries to pass, but falls off from the beam, scoring 2; rat walks on the beam, but the falling number of its injuried posterior limb exceeds 50%, scoring 3; the number is greater than 1, but less than 50%, scoring 4; falling only once, scoring 5; passing smoothly, scoring 6. The rats were trained for 2 days before ischemia to learn how to pass the beam smoothly. The rats were tested respectively on the 3^{rd}, 7^{th}, 10^{th} and 14^{th} day after ischemia.

### 3.4.2 Tactile stimulation test

The somatesthesia and fine motor execution function were evaluated (please give the reference document of such method). Medical tapes of the same area (0.7cm x 0.7cm) were sticked on the ventral side of arm in forelimb respectively and taken as the tactile stimulation to record the latent period of exposing tapes. The rats were trained for 2 days, once per day, before the operation, so that the rats could complete the motion of exposing tapes within 20 seconds. The test was conducted on the 3^{rd}, 7^{th}, 10^{th} and 14^{th} day after ischemia.

### 3.5 Collecting materials

After tests, the rats were anesthetized by intraperitoneal injection of 10% chloral hydrate (0.35 g/kg ), subjected to left ventricle cannula, perfused with 37° physiological saline solution and pre-cooled 4% paraformaldehyde phosphate buffer solution (pH7.2), after the rats were stiff, they were decapitated and their brains were taken out, soaked in 4% paraformaldehyde solution and fixed for 24h, dewatered by conventional method, embedded with paraffin wax, the tissue blocks from 2.2mm to 1.7mm front anterior fontanel were taken, and continuous slicing was conducted at coronal site thereof, the brain slices had a thickness of 3µm.

### 3.6 Histopathologic and immunohistochemical staining

HE staining and immunohistochemical staining (Sp two-steps method) were conducted on 5 cases of each group. Under 200x optical microscope, 3 fixed visual fields were selected around infarct area on each of slices and photoed, and the images were analyzed by using a software, Image-Pro Plus (v.5.1, Silver Spring, Maryland, USA), to observe the morphological structure of nerve cells in cortical motor and sensory areas, and the nerve cells in the 200x visual field (HP) were counted, i.e., n/200 HP; the integral optical density (IOD) and microvascular density (MVD) of the VEGF positive cells (cytoplasm appeared brown particles) in brain tissues around infarct area were measured according to the method of Weidner, et al [Weidner N. et al, N Engl J Med, 1991, 324: 1-8.], any brown-staining endothelial cell or endothelial-cell cluster was considered a single, countable microvessel, and vessel lumens were not necessary for a structure to be defined as a microvessel, to calculate the number of microvessels in area of per m², the average of 3 fields was used as the test result.

### 3.7 Statistic method

SPSS 13.0 statistic software suitable for Windows was used for data statistics and analysis, in which the data were expressed in *x̅* ±s, and the comparison among groups was performed by single factor analysis variance.

### II. Test results

### 1. Effects of the present pharmaceutical composition on motion ans sensory function in cerebral ischemia reperfusion rats

### 1.1 Effects of the present pharmaceutical composition on beam walking ability of cerebral ischemia reperfusion rats

It could be seen in Table 16 that the beam walking ability of the rats of the sham operation group did not change, while the beam walking ability of the rats of ischemia groups decreased significantly, and did not recover to normal level within the observation period. The groups administered with the present pharmaceutical composition at 30 mg/kg, 60 mg/kg, and Angong Niuhuang Wan gave an improved recovery of rat walking ability, and in comparison with the model group, P<0.05, P<0.01, on the 14^{th} day after operation.

**Table 16: Effects of the present pharmaceutical composition on beam walking ability in cerebral ischemia reperfusion rats (x̅ ±s)**

| Group | n | Dose (mg/kg) | Beam walking score | | | |
|---|---|---|---|---|---|---|
| | | | 3day | 7day | 10day | 14day |
| Sham operation | 10 | - | 5.60±0.49 | 6.00±0 | 6.00±0 | 6.00±0 |
| Model | 10 | - | 1.20±0.6 | 1.30±0.46 | 2.10±0.54 | 3.60±0.66# |
| The composition of the present invention (low) | 10 | 15 | 1.00±0.45 | 1.30±0.46 | 1.90±0.70 | 3.90±0.83 |
| The composition of the present invention (middle) | 11 | 30 | 1.00±0.63 | 1.20±0.75 | 2.30±0.46 | 4.27±0.64* |
| The composition of the present invention (high) | 11 | 60 | 1.00±0.60 | 1.30±0.45 | 2.30±0.62 | 4.64±0.77** |
| Angong Niuhuang Wan | 11 | 400 | 1.09±0.51 | 1.40±0.64 | 2.50±0.50 | 4.30±0.45* |

| | | | | | | |
|---|---|---|---|---|---|---|
| Note: in comparison with the model group, * *P*<0.05, * * *P*<0.01; in comparison with the sham operation group, # P<0.01 | | | | | | |

### 1.3 Effects of the present pharmaceutical composition on tactile stimulation response in cerebral ischemia reperfusion rats

The tactile sensitivity and fine motion execution ability of ischemic opposite side forelimbs of rats of ischemia groups weaked significantly, although they are gradually recovered during the observation period, they were still significantly less than that of the normal group after 14 days. In comparison with the model group, the latent periods of exposing tapes of the groups administered with the present pharmaceutical composition of 30mg/kg, 60mg/kg were significantly shortened, P < 0.05.

**Table 17: Effects of the present pharmaceutical composition on tactile stimulation in cerebral ischemia reperfusion rats (x̅ ±s)**

| Group | n | Dose (mg/kg) | Tactile stimulation latent period (s) | | | |
|---|---|---|---|---|---|---|
| | | | 3day | 7day | 10day | 14day |
| Sham operation | 10 | - | 18.80±3.06 | 19.30±3.80 | 18.40±2.37 | 16.10±2.26 |
| Model | 10 | - | 335.20±36.17 | 259.90±28.58 | 146.20±12.39 | 71.70±9.22# |
| The composition of the present invention (low) | 10 | 15 | 317.70±37.05 | 251.90±16.07 | 135.40±19.59 | 73.00±13.75 |
| The composition of the present invention (middle) | 11 | 30 | 328.55±39.56 | 245.64±15.71 | 142.09±16.21 | 60.82±9.97* |
| The composition of the present invention (high) | 11 | 60 | 340.09±31.61 | 231.09±32.71 | 137.64±11.36 | 60.18±8.92* |
| Angong Niuhuang Wan | 11 | 400 | 307.09±38.34 | 232.64±38.51 | 145.00±19.68 | 67.09±7.67 |

| | | | | | | |
|---|---|---|---|---|---|---|
| Note: in comparison with the model group, * P<0.05, * * P<0.01; in comparison with the sham operation group, # P<0.01 | | | | | | |

### 2. Effects of the composition of the present invention on the injury of neurons in cortical motor and sensory areas in cerebral ischemia reperfusion rats

In the cortical motor and sensory areas and neostriatum of rats of the model group, a lot of nerve cells denaturated and dead, arranged disorderedly, and leading to unclear contour profiles of cell membranes and nucleus, nuclear condensation, cell body shrinking, neuron density decrease, apparent neuron missing, losser stroma in sieve shape. In comparison with the model group, the groups administered with the present pharmaceutical composition 30 mg/kg, 60 mg/kg, and the Angong Niuhuang Wan group showed significantly increased neuron number, smaller denaturated and necrotic tissue area, and light extent.

**Table 18: Effects of the present pharmaceutical composition on the neuron number in cortical motor and sensory areas in cerebral ischemia reperfusion rats (x̅ ±s)**

| Group | n | Dose (mg/kg) | n/200x HP |
|---|---|---|---|
| Sham operation | 10 | - | 91.6±8.2 |
| Model | 10 | - | 12.7±4.8# |
| The composition of the present invention (low) | 10 | 15 | 22.8±6.7 |
| The composition of the present invention (middle) | 11 | 30 | 32.2±11.7** |
| The composition of the present invention (high) | 11 | 60 | 47.2±8.4** |
| Angong Niuhuang Wan | 11 | 400 | 39.6±9.8** |

| | | | |
|---|---|---|---|
| Note: in comparison with the model group: * P<0.05, * * P<0.01, in comparison with the sham operation group: # P<0.01 | | | |

### 3. Effects of the composition of the present invention on angiogenesis and VEGF expression in cerebral ischemia reperfusion rats

In the rats of the sham operation group, a small amount of brown microvessels and VEGF-positive neurons and endothelial cells could be seen in their cortexes and striatums; in the rats of the cerebral ischemia groups, a lot of neurons could be seen in areas around cortex and striatum infarct, both glial cells and endothelial cells were of VEGF-positive, scattered or clustered CD34-positive cells and microvessels formed thereby were distributed areas around infarct and extended toward infarct center. In comparison with the model group, the groups administered with the present pharmaceutical composition of 30 mg/kg, 60 mg/kg, and the Angong Niuhuang Wan group showed significantly increased VEGF expression, significantly increased number of microvessels; P<0.05, P<0.01.

**Table 19: Effects of the present pharmaceutical composition on angiogenesis in cerebral ischemia reperfusion rats (x̅ ±s)**

| Group | n | Dose (mg/kg) | MVD(n/mm²) |
|---|---|---|---|
| Sham operation | 5 | - | 214.8±26.8 |
| Model | 5 | - | 359.6±14.5# |
| The composition of the present invention (low) | 5 | 15 | 352.1±37.5 |
| The composition of the present invention (middle) | 5 | 30 | 409.5±28.9* |
| The composition of the present invention (high) | 5 | 60 | 440.4±19.4** |
| Angong Niuhuang Wan | 5 | 400 | 424.5±34.2** |

| | | | |
|---|---|---|---|
| Note: in comparison with the model group: * P<0.05, * * P<0.01; in comparison with the sham operation group: # P<0.01 | | | |

**Table 20: Effects of the present pharmaceutical composition on VEGF expression in cerebral ischemia reperfusion rats (x̅ ±s)**

| Group | n | Dose (mg/kg) | IOD |
|---|---|---|---|
| Sham operation | 5 | - | 70.0±5.4 |
| Model | 5 | - | 172.4±22.8# |
| The composition of the present invention (low) | 5 | 15 | 196.1±26.2 |
| The composition of the present invention (middle) | 5 | 30 | 224.9±26.8* |
| The composition of the present invention (high) | 5 | 60 | 229.9±22.9** |
| Angong Niuhuang Wan | 5 | 400 | 262.2±27.8** |

| | | | |
|---|---|---|---|
| Note: in comparison with the model group: * P<0.05, * * P<0.01; in comparison with the sham operation group: # P<0.01 | | | |

### III. Discussions

After focal brain tissue ischemia reperfusion in rats, the resultant injury leads to denaturation and necrosis of a large amount of nerve cells in cortical motor and sensory areas and neostriatum, limb motor and sensory dysfunction, significant decrease of mortor coordination and balance ability, so that it is a critical step for recoveryof nervous function to establish blood supply for ischemic tissues. In this studying, the composition of the present invention (30 mg/kg, 60 mg/kg) were administered during subacute phase (from the 3^{rd} to 14^{th} day) after ischemia, so that the motor and sensory function of rats were improved significantly. In comparison with the model group, the beam walking ability of rats were improved, the latent period of exposing tapes was shortened, the injury or neurons in cortical motor and sensory areas was alleviated, and the VEGF expression and the number of microvessels in areas around infarct incrased, P<0.05, P<0.01. In sum, the composition of the present invention can accelerate the recovery of motor and sensory function in cerebral ischemia rats, and a possible mechanism thereof is to prompt the expression of brain VEGF and the neogenesis of microvessels.

### Example 5: Preparation of the present pharmaceutical composition

3 Kg of fresh rootstalk of *Rhizome anemarrhenae* was cut into thin slices, added with 8 L of 70% ethanol and soaked for 1 h, extracted under refluxing, filtered, the residue was added with 6L of 70% ethanol and extracted under refluxing twice. The obtained extracting solutions were combined, subjected to ethanol recovery, and concentrated under a reduced pressure to 10 L. Pre-treated macroporous adsorbent resin SP825 (Mitsubishi Company of Japan) was loaded on a column (4L), balanced with water, the concentrated solution was filtered, the filtrate was loaded on the chromatography column, eluted with 4 BV (4 times of the column volume) of water and 4 BV of 20% ethanol to remove impurities, then eluted with 4 BV of 70% ethanol and 3 BV of 95% ethanol, the 70% part of eluting solution was subjected to ethanol recovery, concentrated to 1000 mL, freeze dried to obtain 81 g of primary total saponins. 9 Kg of fresh rootstalk of *Rhizome anemarrhenae* was cut into thin slices, added with 24 L of water, warming with 37°C water-bath for 72h for nat ural fermentation. After filtration, the filtrate was discarded, the residue was extracted with 18 L of methanol under refluxing for 1 h, filtered, the residue was extracted with 18 L of methanol under refluxing for twice in the same way. All methanol extracting solutions were combined, subjected to partial solvent recovery, to obtain a precipitate, the precipitate was dried and weighed to obtain 212 g of crude AIII sample. The measurement of HPLC-ELSD method showed the timosaponin BII content in the primary total saponins was 58.7%, and the timosaponin AIII content in the crude AIII sample was 55.4%. 80 g of the primary total saponins and 20 g of the crude AIII were mixed uniformly to obtain the present pharmaceutical composition. The contents of timosaponin AIII and BII as measured by separately using a HPLC-ELSD external standard two-points method were 40.9% and 16.1 %, and the total timosaponins as measured by an ultraviolet spectrophotometry was 82.7%.

### Example 6: Preparation of present the pharmaceutical composition

6 Kg of decoction pieces of Chinese medicine *Rhizome anemarrhenae* was properly smashed, added with 48 L of water, soaked for 1 h, heated and decocted for 1 h, and filtered; the residue was added with 36 L of water and decocted twice, filtered. The filtrates were combined, and concentrated under a reduced pressure to 30 L, centrifuged to obtain supernatant for standby use. Pre-treated macroporous adsorbent resin SP700 (Mitsubishi Company of Japan) was loaded on a column (18L), balanced with water. The supernatant for standby use was loaded on the balanced SP700 resin column, balanced with water. The concentrated extracting solution was filtered, the filtrate was loaded, washed with water to remove impurities, then eluted in order with 4 BV of 25% ethanol, 4 BV of 90% ethanol. The 90% part of eluting solution was subjected to ethanol recovery, concentrated to 5000 mL. 1000 mL thereof was taken and freeze dried to obtain 78 g of primary total saponins. The residual 4000 mL was diluted by adding water to 15000mL, then added with 20 mL of β-glucosidase, mixed uniformly and placed in 50°C water-bath for 24h for heat preservation and conversion. The converted solution was centrifuged to obtain a supernatant and a precipitate, the precipitate was placed in a drying oven at 80°C for 10h to dry to obtain 213g of secondary total saponins, which was smashed to be of powder form. The contents of timosaponin BII and AIII in the primary total saponins and the secondary total saponins were separately measured by HPLC-ELSD method to be 52.6% and 66.3%. 75 g of the primary total saponins and 180 g of the secondary total saponins were mixed uniformly to obtain the present pharmaceutical composition. The contents of timosaponin AIII and BII as measured by separately using a HPLC-ELSD external standard two-points method were 47.1 % and 15.6%, and the total timosaponins as measured by an ultraviolet spectrophotometry was 88.5%.

### Example 7: Preparation of the present pharmaceutical composition

8 Kg of fibrous root *Rhizome anemarrhenae* was cut, added with 48 L of 65% ethanol, soaked for 1 h, extracted under refluxing for 1 h, and filtered; the residue was added with 48 L of 60% ethanol and extracted under refluxing twice, filtered. The filtrates were combined, subjected to vacuum recovery of ethanol to 20 L, added with ethanol to reach a concentration of 30%, stood for standby use. Pre-treated macroporous adsorbent resin SP700 (Mitsubishi Company of Japan) was loaded on a column (10L), balanced with 30% ethanol. The extracting solution for standby use was centrifuged, the resultant supernatant was loaded on the balanced resin column, eluted in order with 4 BV of 30% ethanol, 4 BV of 90% ethanol. The part of 90% ethanol was collected, subjected to ethanol recovery, concentrated to 4000 mL. 2000 mL thereof was taken and freeze dried to obtain 165 g of primary total saponins. The residual 2000 mL was diluted by adding water to 3200mL, then added with 30 mL of pectinase (NCB-PE40), mixed uniformly and placed in 50°C water-bath for 12h for heat preservation and conversion. The converted solution was centrifuged to obtain a supernatant and a precipitate, the precipitate was placed in a drying oven at 80°C for 6h to dry to obtain 119g of secondary total saponins. The contents of timosaponins BII and AIII in the primary total saponins were measured by HPLC-ELSD method to be 19.2% and 32.6%, and the amount of timosaponin AIII in the secondary total saponins was 61.3%. 100 g of the primary total saponins and 100 g of the secondary total saponins were mixed uniformly to obtain the present pharmaceutical composition. The contents of timosaponin AIII and BII as measured by HPLC-ELSD external standard two-points method were 47.2% and 9.7%, and the total timosaponins as measured by an ultraviolet spectrophotometry was 79.4%.

### Example 8: Preparation of the present pharmaceutical composition

6 Kg of decoction pieces of Chinese medicine *Rhizome anemarrhenae* was properly smashed, added with 48 L of 30% ethanol, soaked for 1 h, extracted under refluxing for 1 h, and filtered; the residue was added with 36 L of 30% ethanol and extracted under refluxing twice in the same way. The ethanol extracting solutions were combined for standby use. Pre-treated macroporous adsorbent resin SP825 (Mitsubishi Company of Japan) was loaded on a column (18L), balanced with 30% ethanol. The supernatant for standby use was loaded on the balanced SP825 resin column, washed with 4 BV of 30% ethanol to remove impurities, then eluted in order with 4 BV of 50% ethanol and 3 BV of 80% ethanol, and the column was regenerated by using 3 BV of 95% ethanol. The eluting solutions of 50% and 80% ethanol were collected, separately subjected to ethanol recovery, vacuum concentration. The 50% ethanol concentrate was freeze dried to obtain 113 g of crude BII, and the 80% ethanol concentrate was dried to obtain 221 g of crude AIII. The contents of timosaponins BII and AIII in the crude BII and the crude AIII as measured by HPLC-ELSD method were 61.2% and 55.9%. 110 g of the crude BII and 180 g of the crude AIII were mixed uniformly to obtain the present pharmaceutical composition. The contents of timosaponin AIII and BII as measured by HPLC-ELSD external standard two-points method were 32.4% and 26.2%, and the total timosaponins as measured by an ultraviolet spectrophotometry was 83.3 %.

### Example 9: Preparation of the present pharmaceutical composition

6 Kg of decoction pieces of Chinese medicine *Rhizome anemarrhenae* was properly smashed, added with 48 L of water, soaked for 1 h, heated and decocted for 1 h, and filtered; the residue was added with 36 L of water and decocted twice in the same way. The extracting solutions were combined, and concentrated under a reduced pressure to 30 L, added with ethanol to reach a concentraton of 30%, shaken uniformly and stood overnight, centrifuged to obtain a supernatant for standby use. Pre-treated macroporous adsorbent resin HP20 (Mitsubishi Company of Japan) was loaded on a column (18L), balanced with 30% ethanol. The supernatant for standby use was loaded on the balanced HP20 resin column, washed with 4BV of 30% ethanol to remove impurities, then eluted in order with 4 BV of 50% ethanol and 4 BV of 80% ethanol, and the column was finally regenerated by using 3BV of 95% ethanol. The 50% and 80% ethanol eluting solutions were collected. The 80% ethanol concentrate was dried to obtain 125 g of crude AIII. The 50% ethanol part was subjected to ethanol recovery, concentrated to 4000 ml. 1500 mL of the 50% ethanol concentrate was taken and freeze dried to obtain 153 g of crude BII. The residual 2500 mL of the solution was diluted by adding water to 7000mL, then added with 40 mL of composite fruit pulp enzyme (NCB-PE200), mixed uniformly and placed in 50°C shaker under 120rpm for 36h for conversion. The converted solution was centrifuged to obtain a precipitate, the precipitate was placed in a drying oven at 80°C for 6h to dry to obtain 183 g of secondary tot al saponins. By measurement of using HPLC-ELSD method, the amount of timosaponin BII in the crude BII was 53.2%, the contents of timosaponin AIII in the crude AIII and the secondary total saponins were 57.1% and 64.3%. 50 g of the crude BII, 120 of the crude AIII and 180 g of the secondary total saponins were mixed uniformly to obtain the present pharmaceutical composition. The contents of timosaponin AIII and BII as measured by HPLC-ELSD external standard two-points method were 52.7% and 7.8%, and the total timosaponins as measured by an ultraviolet spectrophotometry was 85.8%.

### Example 10: Preparation of the present pharmaceutical composition

6 Kg of decoction pieces of Chinese medicine *Rhizome anemarrhenae* was properly smashed, added with 48 L of 50% ethanol, soaked for 1 h, extracted under refluxing for 1 h, and filtered; the residue was added with 36 L of 50% ethanol and extracted under refluxing twice in the same way. The extracting solutions were combined, and concentrated under a reduced pressure to 6 L, added with water-saturated n-butanol for extraction for 3 times, and all n-butanol layers were combined, concentrated to obtain 551 g of primary total saponins. 500 g of the primary total saponins was dissolved in 10000 mL of water, added with 110 mL of cellulase (AE80), mixed uniformly and placed in 50°C shaker under 120rpm for 36h for conversion. The converted solution was centrifuged to obtain a precipitate, the precipitate was placed in a drying oven at 80°C for 6h to dry to obtain 283 g of secondary total saponins. By measurement of using HPLC-ELSD method, the amount of timosaponin BII in the crude BII was 44.1%, the amount of timosaponin AIII in the secondary total saponins was 62.3%. 50 g of the primary total saponins and 280 g of the secondary total saponins were mixed uniformly to obtain the present pharmaceutical composition. The contents of timosaponin AIII and BII as measured by HPLC-ELSD external standard two-points method were 53.7% and 6.9%, and the total timosaponins as measured by an ultraviolet spectrophotometry was 86.1 %.

### Example 11: Preparation of the present pharmaceutical composition

6 Kg of fresh rootstalk of *Rhizome anemarrhenae* was cut into thin slices, added with 8 L of 70% ethanol, soaked for 2 h, extracted for 1 h under heating and refluxing, filtered, the residue was added with 6L of 70% ethanol and extracted in the same way under refluxing twice. The extracting solutions were combined, subjected to ethanol recovery, and concentrated under a reduced pressure to 10 L. Pre-treated macroporous adsorbent resin SP700 (Mitsubishi Company of Japan) was loaded on a column (6L), balanced with 20% ethanol, the concentrated solution was added with ethanol to reach 20%, filtered, the filtrate was loaded on the chromatography column, eluted in order with 4 BV of 20% ethanol, 4 BV of 80% ethanol and 3 BV of 95% ethanol, the 80% ethanol was subjected to ethanol recovery, concentrated to a small volume of 2000 mL. 500 mL thereof was taken and freeze dried to obtain 56 g of primary total saponins. The residual 1500 mL was diluted to 7000 mL, added with 200 mL of emulsin solution, mixed uniformly and placed in a 37°C shaker under 120rpm for 24 h for conversion. The converted solution was centrifuged to obtain a supernatant and a precipitate, the precipitate was placed in a drying oven at 80°C to dry to obtain 105 g of secondary total sapo nins. By measurement of HPLC-ELSD method, the amount of timosaponin BII in the primary total saponins was 43.3%, and the amount of timosaponin AIII in the secondary total saponins was 55.6%. 50 g of the primary total saponins and 100 g of the secondary total saponins were mixed uniformly to obtain the present pharmaceutical composition. The contents of timosaponin AIII and BII as measured by HPLC-ELSD external standard two-points method were 37.5% and 14.7%, and the total timosaponins as measured by an ultraviolet spectrophotometry was 73.5%.

### Example 12: Preparation of the present pharmaceutical composition

6 Kg of fresh rootstalk of *Rhizome anemarrhenae* was cut into thin slices, added with 8 L of 60% ethanol, soaked for 2 h, extracted for 0.5 h under ultrasonic waves using ultrasonic oscillator, filtered, the residue was added with 6L of 60% ethanol and extracted in the same way under ultrasonic waves twice, filtered. The extracting solutions were combined, and concentrated under a reduced pressure to 10 L, added with acetone to reach 20% for standby use. Pre-treated macroporous adsorbent resin AB-8 (Tianjin Nankai Chemical Factory) was loaded on a column (6L), balanced with 20% acetone, the 20% acetone solution for standby use was loaded on the column, eluted in order with 4 BV of 20% acetone, 4 BV of 80% acetone, the 80% acetone part was collected, subjected to acetone recovery, concentrated to 2000 mL. 500 mL thereof was freeze dried to obtain 43 g of primary total saponins. The residual 1500 mL thereof was diluted to 7000 mL, added with 200 mL of emulsin solution, mixed uniformly and placed in a 379°C water-bath for 24 h for heat preservation and conversion. The converted solution was centrifuged to obtain a supernatant and a precipitate, the precipitate was placed in a drying oven at 80°C for 6 h to dry to obtain 94 g of secondary total saponins. By measurement of HPLC-ELSD method, the contents of timosaponin BII and AIII in the primary total saponins and the secondary total saponins were 54.1% and 62.3%, respectively. 43 g of the primary total saponins and 90 g of the secondary total saponins were mixed uniformly to obtain the present pharmaceutical composition. The contents of timosaponin AIII and BII and the total timosaponins were measured separately by using a HPLC-ELSD external standard two-points method and ultraviolet spectrophotometry, in which BII 17.3%, AIII was 42.7%, and total saponins was 83.4%.

### Example 13: Preparation of the present pharmaceutical composition

9 Kg of fresh rootstalk of *Rhizome anemarrhenae* was cut into thin slices, added with 24 L of 40% acetone, soaked for 2 h, extracted for 0.5 h under ultrasonic waves using ultrasonic oscillator, filtered, the residue was added with 18L of 40% acetone and extracted in the same way under ultrasonic waves twice. The extracting solutions were combined, subjected to acetone recovery, and concentrated under a reduced pressure to 10 L. Pre-treated macroporous adsorbent resin D-101 (Tianjin Insecticide Factory) was loaded on a column (12L), balanced with water. The concentrated extracting solution was loaded on the column, eluted in order with 4 BV of water, 4 BV of 15% acetone, 4 BV of 70% acetone, the 70% acetone part was collected, subjected to solvent recovery, concentrated to 3000 mL. 500 mL thereof was freeze dried to obtain 46 g of primary total saponins. The residual 2500 mL thereof was diluted to 11000 mL, added with 54 mL of β-glucanase (NCB-10), mixed uniformly and placed in a 50°C water-bath for 20 h for heat preservation and conversion. The converted solution was centrifuged to obtain a supernatant and a precipitate, the precipitate was placed in a drying oven at 80°C to dry to obtain 163 g of secondary total saponins. By measurement of HPLC-ELSD method, the amount of timosaponin BII in the primary total saponins was 56.2%, and the amount of timosaponin AIII in the secondary total saponins was 63.5%. 40 g of the primary total saponins and 160 g of the secondary total saponins were mixed uniformly to obtain the present pharmaceutical composition. The contents of timosaponin AIII and BII as measured by HPLC-ELSD external standard two-points method were 51.3% and 11.2%, and the total timosaponins as measured by an ultraviolet spectrophotometry was 87.5%.

### Example 14: Preparation of the present pharmaceutical composition

12 Kg of fibrous root of *Rhizome anemarrhenae* was cut, added with 48 L of water, soaked for 1 h, extracted for 0.5 h under ultrasonic waves using ultrasonic oscillator, filtered; the residue was added with 36 L of water and extracted in the same way under ultrasonic waves twice, filtered. The filtrates were combined, and concentrated under a reduced pressure to 20 L, added with ethanol to reach a concentration of 30%, stood for standby use. Pre-treated macroporous adsorbent resin D-101 (Tianjin Insecticide Factory) was loaded on a column (8L), balanced with 30% ethanol. The extracting solution for standby use was centrifuged, the resultant supernatant was loaded on the balanced column, eluted in order with 4 BV of 30% ethanol, 3 BV of 80% ethanol and 3 BV of 95% ethanol, the 80% ethanol part was collected, concentrated to 2000 mL. 400 mL thereof was freeze dried to obtain 21 g of primary total saponins. The residual 1600 mL thereof was added with 2000 mL of *Aspergillus niger* culture solution, mixed uniformly and placed in a 37°C water-bath for 20 h for heat preservation and conversion. The converted solution was centrifuged to obtain a supernatant and a precipitate, the precipitate was placed in a drying oven at 80°C to dry to obtain 63 g of secondary total saponins. By measurement of HPLC-ELSD method, the amount of timosaponin BII in the primary total saponins was 44.3%, and the amount of timosaponin AIII in the secondary total saponins was 52.3%. 20 g of the primary total saponins and 60 g of the secondary total saponins were mixed uniformly to obtain the present pharmaceutical composition. The contents of timosaponin AIII and BII as measured by HPLC-ELSD external standard two-points method were 39.5% and 11.2%, and the total timosaponins as measured by an ultraviolet spectrophotometry was 71.8%.

### Example 15: Preparation of the present pharmaceutical composition

6 Kg of decoction pieces of Chinese medicine *Rhizome anemarrhenae* was properly smashed, added with 48 L of water, soaked for 1 h, heated and decocted for 1 h, and filtered; the residue was added with water and decocted in the same way twice. The extracting solutions were combined, and concentrated under a reduced pressure to 30L, added with ethanol to reach a concentration of 35%, mixed uniformly and stood overnight, centrifuged to obtain a supernatant for standby use and a precipitate which was dried and stored. Pre-treated macroporous adsorbent resin HP20 (Mitsubishi Company of Japan) was loaded on a column (18L), balanced with 35% ethanol. The supernatant for standby use was loaded on the balanced HP20 resin column, washed with 4BV of 35% ethanol to remove impurities, then eluted with 4 BV of 85% ethanol, the column was finally regenerated by using 3 BV of 95% ethanol. The 85% ethanol eluting solution was collected, subjected to ethanol recovery, concentrated to 3000mL. 600 mL of the concentrated solution was freeze dried to obtain 52 g of primary total saponins. The residual 2400 mL of the solution was diluted by adding 1000 mL of acetate buffer solution (pH = 4), mixed uniformly and placed in a 37°C shaker under 120rpm for 24h for conversion. The converted solution was centrifuged to obtain a supernatant and a precipitate, the precipitate was placed in a drying oven at 80°C to dry to obtain 166 g of secondary total saponins. By measurement of using HPLC-ELSD method, the amount of timosaponin BII in the primary total saponins was 50.2%, the amount of timosaponin AIII in the secondary total saponins was 57.1%. 40 g of the primary total saponins and 165 g of the secondary total saponins were mixed uniformly to obtain the present pharmaceutical composition. The contents of timosaponin AIII and BII as measured by HPLC-ELSD external standard two-points method were 46.1% and 10.4%, and the total timosaponins as measured by an ultraviolet spectrophotometry was 81.5%.

### Example 16: Preparation of the present pharmaceutical composition

6 Kg of decoction pieces of Chinese medicine *Rhizome anemarrhenae* was properly smashed, added with 48 L of water, soaked for 1 h, heated and decocted for 1 h, and filtered; the residue was added with water and decocted in the same way twice. The extracting solutions were combined, and concentrated under a reduced pressure to 30L, added with acetone to reach a concentration of 15%, mixed uniformly and stood overnight, centrifuged to obtain a supernatant for standby use. Pre-treated macroporous adsorbent resin SP825 (Mitsubishi Company of Japan) was loaded on a column (18L), balanced with 15% acetone. The supernatant for standby use was loaded on the balanced SP825 resin column, washed with 4BV of 15% acetone to remove impurities, then eluted with 4 BV of 70% acetone. The 70% acetone eluting solution was collected, subjected to solvent recovery, concentrated to 5000m L. 1500 mL of the concentrated solution was freeze dried to obtain 87 g of crude BII. The residual 3500 mL of the solution was added with sulfuric acid to adjust pH to 2-3, mixed uniformly, hydrolyzed and converted for 2h. The converted solution was centrifuged to obtain a supernatant and a precipitate, the precipitate was placed in a drying oven at 80°C to dry to obtain 113 g of secondary to tal saponins. By measurement of using HPLC-ELSD method, the amount of timosaponin BII in the primary total saponins was 55.6%, the amount of timosaponin AIII in the secondary total saponins was 46.3%. The primary total saponins and the secondary total saponins were mixed uniformly to obtain the present pharmaceutical composition. The contents of timosaponin AIII and BII as measured by HPLC-ELSD external standard two-points method were 26.7% and 24.3%, and the total timosaponins as measured by an ultraviolet spectrophotometry was 73.7%.

### Example 17: Preparation of the present pharmaceutical composition

2 Kg of decoction pieces of Chinese medicine *Rhizome anemarrhenae* was properly smashed, added with 16 L of 50% ethanol, soaked for 1 h, extracted under refluxing for 1 h, and filtered; the residue was added with 12 L of 50% ethanol and extracted in the same way twice. The ethanol extracting solutions were combined, subjected to ethanol recovery, and concentrated under a reduced pressure to 10L, to obtain a supernatant for standby use. Pre-treated macroporous adsorbent resin SP700 (Mitsubishi Company of Japan) was loaded on a column (8L), balanced with water. The supernatant for standby use was loaded on the balanced SP700 resin column, washed with 4BV of water and then with 4 BV of 30% ethanol to remove impurities, then eluted with 3 BV of 50% ethanol. The 50% ethanol eluting solution was collected, subjected to ethanol recovery, and concentrated under a reduced pressure to 1500mL. The concentrated solution passed C18 column chromatography repeatedly, eluted with 55% methanol at constant proportion, to finally obtain 32 g of BII (content of percentage area method being greater than 95%). 24 Kg of fresh rootstalk of *Rhizome anemarrhenae* was cut into thin slices, added with 48 L of water, placed in 37°C water-bath for 72 h for heat preservation a nd natural fermentation. After filtration, the filtrate was discarded, the residue was extracted with 48 L of methanol under refluxing for 1 h, filtrated, the residue was further extracted with 48 L of methanol in the same way under refluxing twice. The methanol extracting solutions were combined, subjected to partial solvent recovery to obtain a precipitate, the precipitate was recrystallized with methanol repeatedly to obtain 181 g of a pure product of AIII (content of percentage area method being greater than 95%) (or, the methanol extracting solution was loaded to a silica column, eluted with chloroform-methanol-water system to obtain a pure product of AIII). 20 g of BII and 180 g of AIII were mixed uniformly to obtain the present pharmaceutical composition. The contents of timosaponin AIII and BII in the present pharmaceutical composition as measured by HPLC-ELSD external standard two-points method were 83.7% and 9.2%, and the total timosaponins as measured by an ultraviolet spectrophotometry was 99.4%.

### Example 18: Preparation of the present pharmaceutical composition

6 Kg of decoction pieces of Chinese medicine *Rhizome anemarrhenae* was properly smashed, added with 48 L of 40% ethanol, soaked for 1 h, heated and refluxing for 1 h, and filtered; the residue was added with 36 L of 40% ethanol and refluxed in the same way twice. The extracting solutions were combined, subjected to vacuum ethanol recovery to reach 6 L, then added with ethanol to reach 20%, mixed uniformly and stood overnight, centrifuged to obtain a supernatant for standby use. Pre-treated macroporous adsorbent resin SP700 (Mitsubishi Company of Japan) was loaded on a column (8L), balanced with 20%. The supernatant for standby use was loaded on the balanced SP700 resin column, washed with 4BV of 20% ethanol to remove impurities, then eluted with 3 BV of 90% ethanol. The 90% ethanol eluting solution was collected, subjected to ethanol recovery, and concentrated under a reduced pressure to 1500mL, freeze dried to obtain 498 g of a sample, which was the present pharmaceutical composition. The contents of timosaponin AIII and BII therein as measured by HPLC-ELSD external standard two-points method were 25.3% and 12.5%, and the total timosaponins as measured by an ultraviolet spectrophotometry was 56.4 %.

### Example 19: Preparation of the present pharmaceutical composition

6 Kg of decoction pieces of Chinese medicine *Rhizome anemarrhenae* was properly smashed, added with 48 L of 40% ethanol, soaked for 1 h, heated and refluxing for 1 h, and filtered; the residue was added with 36 L of 40% ethanol and refluxed in the same way twice. The extracting solutions were combined, subjected to vacuum ethanol recovery to reach 6 L, then added with ethanol to reach 25%, mixed uniformly by shaking and stood overnight, centrifuged to obtain a supernatant for standby use. Pre-treated macroporous adsorbent resin SP700 (Mitsubishi Company of Japan) was loaded on a column (8L), balanced with 20%. The supernatant for standby use was loaded on the balanced SP700 resin column, washed with 4BV of 25% ethanol to remove impurities, then eluted with 3 BV of 50% ethanol and 85% ethanol. The 50% and 85% ethanol eluting solutions were collected, subjected to ethanol recovery, vacuum concentrated, and separately freeze dried to obtain 153 g of primary total saponins and 316 g of secondary total saponins. 150 g of the primary total saponins and 300 g of the secondary total saponins were mixed uniformly to obtain the present pharmaceutical composition. The contents of timosaponin AIII and BII in pharmaceutical composition of the present invention as measured by HPLC-ELSD external standard two-points method were 27.6% and 13.5%, and the total timosaponins as measured by an ultraviolet spectrophotometry was 60.3 %.

## Claims

1. A pharmaceutical composition for use in the prophylaxis or treatment of a thrombotic disease, comprising an effective amount of timosaponin AIII and timosaponin BII, and one or more pharmaceutically acceptable excipients, **characterized in that** the amount of timosaponin AIII is greater than or equal to the amount of timosaponin BII,

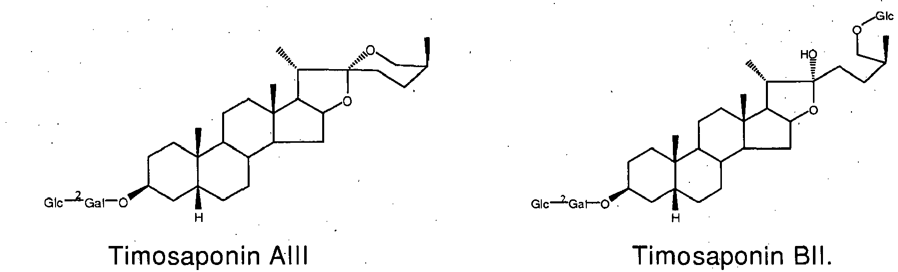

2. A pharmaceutical composition for use in the prophylaxis or treatment of a thrombotic disease, comprising an effective amount of timosaponin AIII. and timosaponin BII, wherein the timosaponin AIII and timosaponin BII are used in the pharmaceutical composition as an extract of timosaponins, **characterized in that** the amount of timosaponin AIII is greater than or equal to the amount of timosaponin BII.

3. The pharmaceutical composition according to claim 1 or 2 for the use according to claim 1 or claim 2, wherein the weight ratio of timosaponin AIII to timosaponin BII is 1:1 to 10:1.

4. The pharmaceutical composition according to claim 1 or 2 for the use according to claim 1 or claim 2, wherein the weight ratio of timosaponin AIII to timosaponin BII is 2:1 to 5:1.

5. The pharmaceutical composition according to claim 1 or 2 for the use according to claim 1 or claim 2, wherein the weight ratio of timosaponin AIII to timosaponin BII is 3:1.

6. The pharmaceutical composition according to claim 1 or 2 for the use according to claim 1 or claim 2, wherein the pharmaceutical composition is formulated in the form of capsules, tablets, granules or an injection.

7. Use of timosaponin AIII and timosaponin BII in the manufacture of a medicament for the prophylaxis or treatment of a thrombotic disease and a thrombus-associated disease, **characterized in that** the amount of timosaponin AIII is greater than or equal to the amount of timosaponin BII in the manufactured medicament.

8. The use according to claim 7, wherein the weight ratio of timosaponin AIII to timosaponin BII is 1:1 to 10:1.

9. The use according to claim 8, wherein the weight ratio of timosaponin AIII to timosaponin BII is 2:1 to 5:1.

10. The use according to claim 8, wherein the weight ratio of timosaponin AIII to timosaponin BII is 3:1.

11. The use according to claim 7, wherein the thrombotic disease is selected from the group consisting of coronary heart disease, angina, myocardial infarction, cerebral apoplexy, cerebral thromobosis, cerebral infarction, pulmonary embolism, diabetes and vasculitis.

12. A method for preparing the pharmaceutical composition according to any one of claims 1 to 6, which comprises the following steps:
extracting decoction pieces, fresh rootstock or fibrous root of *Rhizome anemarrhenae* with 40-70% a C₁-C₄ alcohol or 40-70% acetone, filtering the resultant extracting solution, collecting and centrifuging the filtrate, then loading the supernatant on a macroporous adsorbent resin column, eluting with a solvent selected from water, 20-90% a C₁-C₄ alcohol and 10-80% acetone in a gradient, and collecting the 50-90% C₁-C₄ alcohol component or 35-80% acetone component to obtain primary total saponins of *Rhizome anemarrhenae;*
transforming the components with one or more enzymes selected from the group consisting of β-glucanase, β-glucosidase, pectinase, cellulase, emulsin and *Aspergillus niger* or a microorganism for a sufficient period, and centrifuging the transformed solution to obtain secondary total saponins of *Rhizome anemarrhenae;* and
mixing the primary total saponins and the secondary total saponins of *Rhizome anemarrhenae* in a certain ratio to obtain the present pharmaceutical composition.

13. The method according to claim 12, which comprises the following steps:
extracting decoction pieces, fresh rootstock or fibrous root of *Rhizome anemarrhenae* with 40-70% ethanol, filtering the resultant extracting solution and collecting the filtrate, concentrating under a reduced pressure and then adding with 90-100% ethanol, centrifuging, then loading the supernatant on a macroporous adsorbent resin column, eluting with 20-95% ethanol in a gradient, and collecting the 50-90% ethanol component to obtain primary total saponins of *Rhizome anemarrhenae;*
transforming the components with one or more enzymes selected from the group consisting of β-glucanase, β-glucosidase, pectinase, cellulase, emulsin and Aspergillus niger or a microorganism for at least one hour, and centrifuging the transformed solution to obtain secondary total saponins of *Rhizome anemarrhenae;* and
mixing the primary total saponins and the secondary total saponins of *Rhizome anemarrhenae* in a certain ratio to obtain the present pharmaceutical composition.

14. A pharmaceutical composition comprising an effective amount of timosaponin AIII and timosaponin BII, and one or more pharmaceutically acceptable excipients, wherein the amount of timosaponin AIII is greater than or equal to the amount of timosaponin BII, for use in the prophylaxis or treatment of a thrombotic disease.

## Patentansprüche

1. Pharmazeutische Zusammensetzung zur Verwendung bei der Vorbeugung oder Behandlung einer Thromboseerkrankung, die eine wirksame Menge an Timosaponin AIII und Timosaponin BII und einen oder mehrere pharmazeutisch unbedenkliche Exzipienten umfasst, **dadurch gekennzeichnet, dass** die Menge an Timosaponin AIII höher als die Menge an Timosaponin BII oder gleich dieser ist,

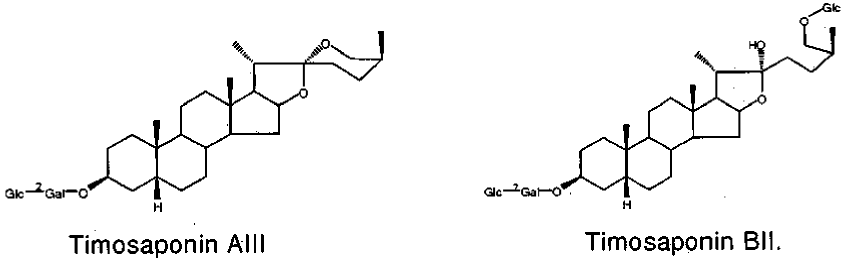

2. Pharmazeutische Zusammensetzung zur Verwendung bei der Vorbeugung oder Behandlung einer Thromboseerkrankung, die eine wirksame Menge an Timosaponin AIII und Timosaponin BII umfasst, wobei das Timosaponin AIII und Timosaponin BII in der pharmazeutischen Zusammensetzung als Timosaponinextrakt verwendet werden, **dadurch gekennzeichnet, dass** die Menge an Timosaponin AIII höher als die Menge an Timosaponin BII oder gleich dieser ist.

3. Pharmazeutische Zusammensetzung nach Anspruch 1 oder 2 zur Verwendung nach Anspruch 1 oder 2, wobei das Gewichtsverhältnis von Timosaponin AIII zu Timosaponin BII 1:1 bis 10:1 ist.

4. Pharmazeutische Zusammensetzung nach Anspruch 1 oder 2 zur Verwendung nach Anspruch 1 oder 2, wobei das Gewichtsverhältnis von Timosaponin AIII zu Timosaponin BII 2:1 bis 5:1 ist.

5. Pharmazeutische Zusammensetzung nach Anspruch 1 oder 2 zur Verwendung nach Anspruch 1 oder 2, wobei das Gewichtsverhältnis von Timosaponin AIII zu Timosaponin BII 3:1 ist.

6. Pharmazeutische Zusammensetzung nach Anspruch 1 oder 2 zur Verwendung nach Anspruch 1 oder 2, wobei die pharmazeutische Zusammensetzung in Form von Kapseln, Tabletten, Granulaten oder einer Injektion formuliert ist.

7. Verwendung von Timosaponin AIII und Timosaponin BII bei der Herstellung eines Medikaments zur Vorbeugung oder Behandlung einer Thrombosererkrankung und einer thrombusassoziierten Erkrankung, **dadurch gekennzeichnet, dass** die Menge an Timosaponin AIII im hergestellten Medikament höher als die Menge an Timosaponin BII oder gleich dieser ist.

8. Verwendung nach Anspruch 7, wobei das Gewichtsverhältnis von Timosaponin AIII zu Timosaponin BII 1:1 bis 10:1 ist.

9. Verwendung nach Anspruch 8, wobei das Gewichtsverhältnis von Timosaponin AIII zu Timosaponin BII 2:1 bis 5:1 ist.

10. Verwendung nach Anspruch 8, wobei das Gewichtsverhältnis von Timosaponin AIII zu Timosaponin BII 3:1 ist.

11. Verwendung nach Anspruch 7, wobei die Thromboseerkrankung aus der Gruppe ausgewählt ist, bestehend aus koronarer Herzkrankheit, Angina, Myokardinfarkt, zerebraler Apoplexie, zerebraler Thrombose, Hirninfarkt, Lungenembolie, Diabetes und Vaskulitis.

12. Verfahren zum Herstellen der pharmazeutischen Zusammensetzung nach einem der Ansprüche 1 bis 6, das die folgenden Schritte umfasst:
Extrahieren von Dekoktstücken, frischem Wurzelstock oder Faserwurzeln von *Rhizome anemarrhenae* mit einem 40%igen bis 70%igem C₁-C₄-Alkohol oder 40%igen bis 70%igem Aceton, Filtern der entstehenden Extraktionslösung, Sammeln und Zentrifugieren des Filtrats, danach Laden des Überstands auf eine makroporöse adsorbierende Harzsäule, Eluieren mit einem Lösungsmittel, das aus Wasser, einem 20%igen bis 90%igem C₁-C₄-Alkohol und 10%igem bis 80%igem Aceton ausgewählt ist, in einem Gradienten und Sammeln der 50%igen bis 90%igen C₁-C₄-Alkoholkomponente oder 35%igen bis 80%igen Acetonkomponente, um primäre Gesamtsaponine von *Rhizome anemarrhenae* zu erhalten;
Umwandeln der Komponenten mit einem oder mehreren Enzymen, die aus der Gruppe ausgewählt sind, bestehend aus β-Glukanase, β-Glukosidase, Pektinase, Zellulase, Emulsin und *Aspergillus niger*, oder einem Mikroorganismus für einen ausreichenden Zeitraum, und Zentrifugieren der umgewandelten Lösung, um sekundäre Gesamtsaponine von *Rhizome anemarrhenae* zu erhalten; und
Mischen der primären Gesamtsaponine und der sekundären Gesamtsaponine von *Rhizome anemarrhenae* in einem gewissen Verhältnis, um die vorliegende Zusammensetzung zu erhalten.

13. Verfahren nach Anspruch 12, das die folgenden Schritte umfasst:
Extrahieren von Dekoktstücken, frischem Wurzelstock oder Faserwurzeln von *Rhizome anemarrhenae* mit 40%igen bis 70% Ethanol, Filtern der entstehenden Extraktionslösung und Sammeln des Filtrats, Konzentrieren unter verringertem Druck und danach Hinzufügen mit 90%igem bis 100%igem Ethanol, Zentrifugieren, danach Laden des Überstands auf eine makroporöse adsorbierende Harzsäule, Eluieren mit 20%igem bis 95%igem Ethanol in einem Gradienten und Sammeln der 50%igen bis 90%igen Ethanolkomponente, um primäre Gesamtsaponine von *Rhizome anemarrhenae* zu erhalten;
Umwandeln der Komponenten mit einem oder mehreren Enzymen, die aus der Gruppe ausgewählt sind, bestehend aus β-Glukanase, β-Glukosidase, Pektinase, Zellulase, Emulsin und Aspergillus niger, oder einem Mikroorganismus für zumindest eine Stunde und Zentrifugieren der umgewandelten Lösung, um sekundäre Gesamtsaponine von *Rhizome anemarrhenae* zu erhalten; und
Mischen der primären Gesamtsaponine und der sekundären Gesamtsaponine von *Rhizome anemarrhenae* in einem gewissen Verhältnis, um die vorliegende Zusammensetzung zu erhalten.

14. Pharmazeutische Zusammensetzung, die eine wirksame Menge an Timosaponin AIII und Timosaponin BII und einen oder mehrere pharmazeutisch unbedenkliche Exzipienten umfasst, wobei die Menge an Timosaponin AIII höher als die Menge an Timosaponin BII oder gleich dieser ist, zur Verwendung bei der Vorbeugung oder Behandlung einer Thromboseerkrankung.

## Revendications

1. Composition pharmaceutique pour utilisation dans la prophylaxie ou le traitement d'une maladie thrombotique, comprenant une quantité efficace de timosaponine AIII et de timosaponine BII, et un ou plusieurs excipients pharmaceutiquement acceptables, **caractérisée en ce que** la quantité de timosaponine AIII est supérieure ou égale à la quantité de timosaponine BII,

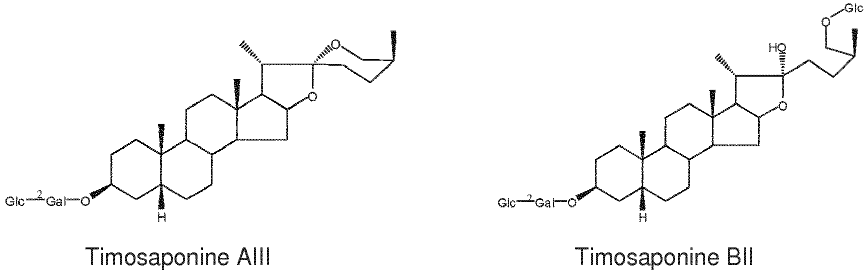

2. Composition pharmaceutique pour utilisation dans la prophylaxie ou le traitement d'une maladie thrombotique, comprenant une quantité efficace de timosaponine AIII et de timosaponine BII, dans laquelle la timosaponine AIII et la timosaponine BII sont utilisées dans la composition pharmaceutique sous la forme d'un extrait de timosaponines, **caractérisée en ce que** la quantité de timosaponine AIII est supérieure ou égale à la quantité de timosaponine BII.

3. Composition pharmaceutique selon la revendication 1 ou 2, pour utilisation selon la revendication 1 ou 2, dans laquelle le rapport en poids de la timosaponine AIII à la timosaponine BII est de 1/1 à 10/1.

4. Composition pharmaceutique selon la revendication 1 ou 2, pour utilisation selon la revendication 1 ou 2, dans laquelle le rapport en poids de la timosaponine AIII à la timosaponine BII est de 2/1 à 5/1.

5. Composition pharmaceutique selon la revendication 1 ou 2, pour utilisation selon la revendication 1 ou 2, dans laquelle le rapport en poids de la timosaponine AIII à la timosaponine BII est de 3/1.

6. Composition pharmaceutique selon la revendication 1 ou 2, pour utilisation selon la revendication 1 ou 2, laquelle composition pharmaceutique est formulée sous la forme de capsules, de comprimés, de granules, ou d'une composition injectable.

7. Utilisation de timosaponine AIII et de timosaponine BII dans la fabrication d'un médicament destiné à la prophylaxie ou au traitement d'une maladie thrombotique et d'une maladie associée à un thrombus, **caractérisée en ce que** la quantité de timosaponine AIII est supérieure ou égale à la quantité de timosaponine BII dans le médicament fabriqué.

8. Utilisation selon la revendication 7, dans laquelle le rapport en poids de la timosaponine AIII à la timosaponine BII est de 1/1 à 10/1.

9. Utilisation selon la revendication 8, dans laquelle le rapport en poids de la timosaponine AIII à la timosaponine BII est de 2/1 à 5/1.

10. Utilisation selon la revendication 8, dans laquelle le rapport en poids de la timosaponine AIII à la timosaponine BII est de 3/1.

11. Utilisation selon la revendication 7, dans laquelle la maladie thrombotique est choisie dans le groupe constitué par une maladie coronarienne, une angine de poitrine, un infarctus du myocarde, une apoplexie cérébrale, une thrombose cérébrale, un infarctus cérébral, une embolie pulmonaire, un diabète, et une angéite.

12. Procédé pour préparer la composition pharmaceutique selon l'une quelconque des revendications 1 à 6, qui comprend les étapes suivantes :
extraction de fragments de décoction, de rhizomes frais ou de racines fibreuses de *Rhizome anemarrhenae* avec 40 à 70 % d'un alcool en C₁ à C₄ ou 40 à 70 % d'acétone, filtration de la solution d'extraction résultante, collecte et centrifugation du filtrat, puis chargement du surnageant sur une colonne de résine adsorbante macroporeuse, élution avec un solvant choisi parmi l'eau, 20 à 90 % d'un alcool en C₁ à C₄ et 10 à 80 % d'acétone dans un gradient, et collecte des 50 à 90 % de composant alcool en C₁ à C₄ ou des 35 à 80 % de composant acétone pour obtenir des saponines totales primaires de *Rhizome anemarrhenae ;*
transformation des composants avec une ou plusieurs enzymes choisies dans le groupe constitué par la β-glucanase, la β-glucosidase, la pectinase, la cellulase, l'émulsine et *Aspergillus niger* ou un microorganisme pendant une période suffisante, et centrifugation de la solution transformée pour obtenir des saponines totales secondaires de *Rhizome anemarrhenae ;* et
mélange des saponines totales primaires et des saponines totales secondaires de *Rhizome anemarrhenae* en une certaine proportion pour obtenir la présente composition pharmaceutique.

13. Procédé selon la revendication 12, qui comprend les étapes suivantes :
extraction de fragments de décoction, de rhizomes frais ou de racines fibreuses de *Rhizome anemarrhenae* avec 40 à 70 % d'éthanol, filtration de la solution d'extraction résultante et collecte du filtrat, concentration sous pression réduite et ensuite addition de 90 à 100 % d'éthanol, centrifugation, puis chargement du surnageant sur une colonne de résine adsorbante macroporeuse, élution avec 20 à 95 % d'éthanol dans un gradient, et collecte des 50 à 90 % de composant éthanol pour obtenir des saponines totales primaires de *Rhizome anemarrhenae ;*
transformation des composants avec une ou plusieurs enzymes choisies dans le groupe constitué par la β-glucanase, la β-glucosidase, la pectinase, la cellulase, l'émulsine et *Aspergillus niger* ou un microorganisme pendant au moins une heure, et centrifugation de la solution transformée pour obtenir des saponines totales secondaires de *Rhizome anemarrhenae ;* et
mélange des saponines totales primaires et des saponines totales secondaires de *Rhizome anemarrhenae* en une certaine proportion pour obtenir la présente composition pharmaceutique.

14. Composition pharmaceutique comprenant une quantité efficace de timosaponine AIII et de timosaponine BII, et un ou plusieurs excipients pharmaceutiquement acceptables, dans laquelle la quantité de timosaponine AIII est supérieure ou égale à la quantité de timosaponine BII, pour utilisation dans la prophylaxie ou le traitement d'une maladie thrombotique.
